# EUROPEAN PATENT APPLICATION

(11) **EP 3 842 526 A1**
(43) Date of publication of application: **30.06.2021**
(21) Application number: 19851814.4
(22) Date of filing: 20.08.2019
(51) Int. Cl.: C12N 5/10, C12N 5/078, C12N 15/09, C12M 3/06

(54) **METHOD FOR CULTIVATING OR INDUCING CELLS**

(30) Priority: 20.08.2018 US 201862766598 P
(71) Applicant: I Peace, Inc., Palo Alto, CA 94303 (US)
(72) Inventor: SUTO, Kenta, Palo Alto, California 94303 (US); TANABE, Koji, Palo Alto, California 94303 (US)
(74) Representative: Zacco Sweden AB
(86) International application number: PCT/JP2019/032438
(87) International publication number: WO 2020/040135

(57) **Abstract**

Provided is a cell culture or induction method including subjecting the cells to culture or induction in a sealed system.

## Description

### [Technical Field]

The present invention relates to cell techniques and relates to culture or induction method of cells.

### [Background Art]

Embryonic stem cells (ES cells) are stem cells established from early embryos of human and mouse. ES cells have pluripotency of differentiation to any cells present in an organism. Currently, human ES cells are available for cell transplantation therapy for many diseases, such as Parkinson's disease, juvenile diabetes, and leukemia. However, transplantation of ES cells still has some obstacles. In particular, transplantation of ES cells can trigger immune rejection similar to the rejection that occurs following unsuccessful organ transplantation. There are also many criticisms and disagreements from an ethical standpoint regarding the use of ES cells established by destroying human embryos.

Against such background, Professor Shinya Yamanaka of Kyoto University succeeded in establishing induced pluripotent stem cell (iPS cells) by introducing four genes: OCT3/4, KLF4, c-MYC and SOX2 into somatic cells. As a result, Professor Yamanaka received the Nobel Prize for Physiology and Medicine in 2012 (see, for example, Patent Documents 1 and 2). iPS cells are ideal pluripotent cells without rejection or ethical problems. Therefore, iPS cells are expected to be used in cell transplantation therapy.

### [Citation List]

### [Patent Literature]

[Patent Literature 1] JP 4183742B
[Patent Literature 2] JP 2014-114997A

### [Summary of Invention]

### [Technical Problem]

A method capable of efficiently and conveniently culturing or inducing various cells, not limited to iPS cells, is desired. Accordingly, it is an object of the present invention to provide a method capable of efficiently and conveniently culturing or inducing cells.

### [Solution to Problem]

According to an aspect of the present invention, there is provided a cell culture or induction method including subjecting the cells to culture or induction in a sealed system.

In the above method, induction may include at least any of reprogramming, initialization, differentiation transformation, differentiation induction, and cell fate reprogramming.

In the above method, there is no need for exchanging gases between the inside and the outside of the sealed system.

The above method may further include controlling the temperature within the sealed system

In the culturing of the above method, the sealed system may be sealed up.

In the above method, it is unnecessary for the outside air to enter the sealed system while the sealed system is sealed up.

In the above method, cells, microorganisms, viruses, and dust outside the sealed system may not enter the sealed system while the sealed system is sealed up.

In the above method, it is not necessary that a material in the sealed system does not flow out of the sealed system while the sealed system is sealed up.

In the above method, at least one of carbon dioxide gas, nitrogen gas, and oxygen gas may not be supplied into the sealed system.

In the above method, the pH of culture medium in the sealed system may be kept within a predetermined range.

In the above method, at least a portion of the sealed system may be formed by being embedded in a gas impermeable material.

In the above method, at least a portion of the sealed system may consist of a gas impermeable material.

In the above method, cells may be cultured or induced while supplementing or replacing culture medium in the sealed system.

In the above method, cells may be cultured or induced while circulating culture medium in the sealed system.

In the above method, the sealed system may include a culture chamber for culturing cells, the culture chamber being provided with a feed port for supplying a fluid into the culture chamber and a discharge port for discharging a fluid in the sealed system, wherein the feed port and the discharge port may be sealable.

In the above method, a feeder for supplying fluid to the feed port is detachable; a discharger for discharging fluid to the discharge port is detachable; and the fluid in culture chamber may be moved into the discharger when the fluid is supplied from the feeder into the culture chamber.

In the above method, when culture medium is supplied from the feeder into the culture chamber, air in the culture chamber may move into the discharger.

In the above method, when culture medium is supplied from the feeder into the culture chamber, the culture medium in the culture chamber may be moved into the discharger.

In the above method, the culture medium may contain cells.

In the above method, when the fluid is supplied from the feeder into the culture chamber, it is not necessary that the outside air does not enter into the culture chamber.

In the above method, in the culturing, the carbon dioxide concentration in the sealed system may not be controlled.

In the culturing of the above method, the carbon dioxide concentration outside the sealed system may not be controlled.

In the culturing of the above method, the material in the sealed system may be transferred via a semipermeable membrane in the sealed system.

In the above method, the sealed system includes a culture chamber for culturing cells and a channel connected to the culture chamber, and the culture medium may circulate through the channel and the culture chamber.

In the above method, gas exchange may not occur with the outside in the channel.

In the above method, the pH of the culture medium in the culture chamber may be kept within a predetermined range by circulation of the culture medium.

In the above method, the culture may be floating culture.

In the above method, the culture may be adherent culture.

In the above method, cells may be cultured in gel culture medium in the sealed system.

In the above method, cells may be cultured in liquid medium in the sealed system.

In the above method, the culture medium in the sealed system may be agitated.

In the above method, the culture medium in the sealed system may not be agitated.

The above method may further include passaging the cells.

In the above method, culture medium may not be added or replaced between seeding and passage.

In the above method, culture medium may be added or replaced between seeding and passage.

In the above method, no culture medium may be added or replaced between passages.

In the above method, culture medium may be added or replaced between passages.

In the above method, the cell may be a stem cell.

In the above method, the stem cell may be an iPS cell, an ES cell, or a somatic stem cell.

In the culturing of the above method, the stem cells may remain undifferentiated.

In the above method, in the culturing, the stem cell may maintain pluripotency.

In the above method, the cell may be a somatic cell.

In the above method, the cell may be at least one selected from the group consisting of: blood cell; nerve cell; cardiac muscle system cell; epithelial cell; mesenchymal cell; hepatocyte; insulin producing cell; retinal pigment epithelial cell; and corneal cell.

In the above method, the cell may be a cell into which an inducing factor has been introduced.

In the above method, the inducing factor may be added to the culture medium in the sealed system and the inducing factor may be introduced into the cells cultured in the sealed system.

In the above method, the cell may be derived from a stem cell.

In the above method, the stem cell may be an iPS cell.

In the above method, the cell may be a blood cell.

In the above method, the cell may be induced into another type of cell.

In the above method, the cell may be a blood cell wherein an inducing factor may be added to the culture medium in the sealed system, an inducing factor may be introduced into the blood cell cultured in the sealed system, and the blood cell may be induced into iPS cells.

In the above method, the inducing factor may be included in an plasmid.

In the above method, the inducing factor may be an RNA.

In the above method, the inducing factor may be included in a Sendai virus.

In addition, according to an aspect of the present invention, there is provided a culture or induction method of cells including: preparing a cell culture vessel having a culture component permeable member through which culture component is permeable, a culture chamber for culturing cells covering one surface of the culture component permeable member and holding a culture medium containing cells, and a culture medium holding chamber for holding culture medium covering the other surface of the culture component permeable member; and culturing or inducing the cells in the culture chamber.

In the above method, the inducing may include at least one of: reprogramming, initialization, differentiation transformation, differentiation induction, and cell fate reprogramming.

In the above method, the interior of the cell culture vessel may be sealed up from the outside.

In the above method, the pH of the culture medium in the cell culture vessel may be kept within a predetermined range.

In the above method, the culture may be floating culture.

In the above method, the cell may be a stem cell.

In the above method, the cell may be a somatic cell.

In the above method, the cell may be at least one selected from the group consisting of: blood cell; nerve cell; cardiac muscle system cell; epithelial cell; mesenchymal cell; hepatocyte; insulin producing cell; retinal pigment epithelial cell; and corneal cell.

In the above method, the cell may be a cell into which an inducing factor has been introduced.

In the above method, the inducing factor may be added to the culture medium in the culture chamber and the inducing factor may be introduced into the cells cultured in the culture chamber.

In the above method, the cell may be induced into another type of cell.

In the above method, the cell culture vessel may further include a culture side plate provided with an opening, which is superposed on the culture chamber side surface of the culture component permeable member.

In the above method, the cell culture vessel may further include a culture medium side plate provided with an opening, which is superposed on the culture medium holding chamber side surface of the culture component permeable member.

In the above method, the culture side plate may be dark colored.

The above method may further include observing cells or a cell mass consisting of cells against a background of a portion of the culture side plate where no opening is provided.

The above method may further include photographing cells or a cell mass consisting of cells against a background of a portion of the culture side plate where no opening is provided.

The above method may further include supplementing or replacing culture medium of the culture medium holding chamber.

### [Advantageous Effects of the Invention]

According to the present invention, it is possible to provide a method capable of efficiently and conveniently culturing or inducing cells.

### [Brief Description of Drawings]

[Fig.1] Fig.1 is an exploded perspective view of a cell culture vessel according to the embodiment.
[Fig.2] Fig.2 is a perspective view of the cell culture vessel according to the embodiment.
[Fig.3] Fig.3 is a front view of a portion of the cell culture vessel according to the embodiment.
[Fig.4] Fig.4 is a perspective view of a part of the cell culture vessel according to the embodiment.
[Fig.5] Fig.5 is a front view of a portion of the cell culture vessel according to the embodiment.
[Fig.6] Fig.6 is a back view of the cell culture vessel according to the embodiment.
[Fig.7] Fig.7 is an exploded perspective view of the cell culture vessel according to the embodiment.
[Fig.8] Fig.8 is a light micrograph of cells cultured in the culturing method according to Example 1.
[Fig.9] Fig.9 is a histogram showing the results of an analysis of the cells cultured in the culturing method according to Example 1 by flow cytometry.
[Fig. 10] Fig.10(a) is a photograph of a tube used in the culturing method according to Example 2.
Fig. 10(b) is a light micrograph of cells cultured in the culturing method according to Example 2.
[Fig. 11] Fig.11 is a histogram showing the results of an analysis of the cells cultured in the culturing method according to Example 2 by flow cytometry.
[Fig. 12] Fig.12 is an optical microscope photograph of cells cultured in the culturing method according to Example 3.
[Fig.13] Fig.13 is a histogram showing the results of an analysis of the cells cultured in the culturing method according to Example 3 by flow cytometry.
[Fig.14] Fig.14 is a photograph of a flask used in a production method of artificial pluripotent stem cells according to Example 4.
[Fig. 15] Fig.15 is an optical micrograph of cells produced by the production method of artificial pluripotent stem cells according to Example 4.
[Fig.16] Fig.16 is a graph showing the number of colonies of cells produced by the production method of artificial pluripotent stem cells according to Example 4 for each colony morphology.
[Fig.17] Fig.17 is a histogram showing the results of an analysis of the cells produced by the production method of artificial pluripotent stem cells according to Example 4 by flow cytometry.
[Fig.18] Fig.18 is an optical micrograph of cells produced in a production method of artificial pluripotent stem cells according to Example 5.
[Fig.19] Fig.19 is a histogram showing the results of an analysis of the cells produced in the production method of artificial pluripotent stem cells according to Example 5 by flow cytometry.
[Fig.20] Fig.20 is an exploded perspective view of a cell culture vessel according to Example 6.
[Fig.21] Fig.21 is a perspective view of the cell culture vessel according to Example 6.
[Fig.22] Fig.22 is a micrograph of a cell mass according to Example 6.
[Fig.23] Fig.23 is a histogram showing the results of flow cytometry of the iPS cells according to Example 6.
[Fig.24] Fig.24 is a micrograph of a cell mass according to Example 7.
[Fig.25] Fig.25 is a histogram showing the results of flow cytometry of the iPS cells according to Example 7.
[Fig.26] Fig.26 is a micrograph of a cell mass according to Example 8.
[Fig.27] Fig.27 is a histogram showing the results of flow cytometry of the iPS cells according to Example 8.

### [Description of Embodiments]

A method of culturing a cell according to an embodiment includes culturing a cell in a sealed system The sealed system is, for example, a completely sealed system in which no exchanges of gases take place between the inside and the outside of the sealed system. The sealed system is, for example, sealed up and does not allow outside air to enter the sealed system. For example, cells, microorganisms, viruses, and dust outside the sealed system do not enter the sealed system. For example, material in the sealed system does not flow out of the sealed system.

Cells may be cultured in a liquid medium in the sealed system or may be cultured in a gel culture medium. Also, the cells may be subjected to adherent culture or floating culture in the sealed system While culturing cells in the sealed system, the culture medium may or may not be agitated. When the cells are subjected to adherent culture, feeder cells may be used or feeder cells may not be used. Feeder cells may not be used when the cells are subjected to floating culture.

The cells cultured in the sealed system may be animal cells including humans cells, insect cells, or plant cells.

The cells cultured in the sealed system may be, for example, somatic cells, differentiated cells, undifferentiated cells, or stem cells. The cells cultured in the sealed system are not particularly limited, and may be, for example, blood cells, nerve cells, cardiac muscle system cells, epithelial cells, vascular endothelial cells, mesenchymal cells, fibroblasts, hepatocytes, insulin producing cells, retinal pigment epithelial cells, and corneal cells.

The blood cells may be hemocytes such as T cells, B cells, NK cells, NKT cells, megakaryocytes, macrophages, granulocytes, neutrophils, eosinophils, hematopoietic stem cells, blood stem/progenitor cells, red blood cells, white blood cells, and platelets. The nerve cells may be neurons and glial cells, oligodendrocytes, or neural stem cells. The cardiac muscle system cells may be myocardial stem cells and myocardial cells, or pacemaker cells. The epithelial cells may be keratinocytes, intestinal epithelial cells, oral epitheliums, or corneal epithelial cells. The mesenchymal cells may be dermal cells, osteoblasts, adipocytes, myocytes, chondrocytes, and the like.

The stem cells are, for example, artificial pluripotent stem (iPS) cells, embryonic stem cells (ES cells), and somatic stem cells. The somatic stem cells may be mesenchymal stem cells. When the cells are stem cells, the stem cells will proliferate in the culture medium while maintaining undifferentiated state and pluripotency.

For example, the stem cells are broken down into single cells or cell masses prior to floating culture, and stem cells broken down into single cells or cell masses are placed in a culture medium. Single cells or cell masses grow while retaining clonality and form colonies in the culture medium.

The stem cells are cultured, for example, in a stem cell culture medium. As culture medium for stem cells, for example, a human ES/iPS culture medium such as mTeSR1 (R) (STEMCELL TECHNOLOGIES) or the like can be used.

However, the culture medium for stem cells is not limited to this, and various stem cells culture medium can be used. For example, as a stem cell culture medium, a culture medium comprising 20%KnockOut SR(R) (ThermoFisher SCIENTIFIC), GlutaMAX(R) (ThermoFisher SCIENTIFIC), and non-essential amino acids (NEAA) may be used. Alternatively, as stem cell culture medium, Primate ES Cell Medium, Reprostem, ReproFF, ReproFF2, ReproXF (Reprocell), TeSR2, TeSRE8, ReproTeSR (STEMCELL Technologies), PluriSTEM(R) Human ES/iPS Medium (Merck), NutriStem(R) XF/FF Culture Medium for Human iPS and ES Cells, Pluriton reprogramming medium (Stemgent), PluriSTEM(R), Stemfit AK02N, Stemfit AK03 (Ajinomoto), ESC-Sure(R) serum and feeder free medium for hESC/iPS (Applied StemCell), and L7(R) hPSC Culture System (LONZA) may be used.

The culture medium in the sealed system is appropriately selected depending on the type of cells to be cultured in the sealed system. For example, when the cells are blood cells, a culture medium suitable for blood cells is placed in the sealed system. For example, when the cells are mesenchymal cells, a culture medium suitable for mesenchymal cells is placed in the sealed system.

The culture medium may be free of growth factor, such as, basic fibroblast growth factor (bFGF). Alternatively, the culture medium may comprise a growth factor such as bFGF at a low concentration of 400 µg/L or less, 40 µg/L or less, or 10 µg/L or less.

Further, the culture medium may not contain tgf-β. Alternatively, the culture medium may contain tgf-β at a low concentration of 2 µg/L (2 ng/mL) or less, 600 ng/L or less, 300 ng/L or less, or 100 ng/L or less.

The culture medium may include at least one material selected from the group consisting of cadherin, laminin, fibronectin, and vitronectin.

When the culture medium is a gel culture medium, the gel culture medium is prepared, for example, by adding deacylated gellan gum to the culture medium such that the final concentration is from 0.001% by weight to 0.5% by weight, from 0.005% by weight to 0.1% by weight, or from 0.01% by weight to 0.05% by weight.

The gel culture medium may include at least one polymeric compound selected from the group consisting of gellan gum, hyaluronic acid, ramsan gum, diutan gum, xanthan gum, carrageenan, fucoidan, pectin, pectic acid, pectinic acid, heparan sulfate, heparin, heparin sulfate, kerato sulfate, chondroitin sulfate, deltaman sulfate, ramnan sulfate, and salts thereof. Further, the gel culture medium may contain methylcellulose. By containing methylcellulose, aggregation between cells is further suppressed.

Alternatively, the gel culture medium may include at least one temperature-sensitive gel selected from: poly(glycerol monomethacrylate) (PGMA), poly(2-hydroxypropyl methacrylate) (PHPMA), Poly(N-isopropylacrylamide) (PNIPAM), amine terminated, carboxylic acid terminated, maleimide terminated, N-hydroxysuccinimide (NHS) ester terminated, triethoxysilane terminated, Poly(N-isopropylacrylamide-co-acrylamide, Poly(N-isopropylacrylamide-co-acrylic acid, Poly(N-isopropylacrylamide-co-butylacrylate, Poly(N-isopropylacrylamide-co-methacrylic acid, Poly(N-isopropylacrylamide-co-methacrylic acid-co-octadecyl acrylate, and N-Isopropylacry lamide.

It is to be noted that, in the present disclosure, a gel-like culture medium or gel culture medium encompasses polymer culture medium.

During culture of the cells in the sealed system, the temperature of the culture medium in the sealed system is kept at, for example, 0 °C or higher, 4 °C or higher, 15 °C or higher, 20 °C or higher, or 34 °C or higher. Further, during culture of the cells in the sealed system, the temperature of the culture medium in the sealed system is kept at, for example, 45 °C or less, 39 °C or less, or 20 °C or less. Temperature control devices, such as heaters and coolers, may be used to control the temperature of the culture medium in the sealed system during the cells are being cultured in the sealed system.

The pH of the culture medium contained in the sealed system is, for example, 4.0 or more, 5.0 or more, 6.0 or more, 7.0 or more, or 8.0 or more. The pH of the culture medium contained in the sealed system is, for example, 10.0 or less, 9.0 or less, 8.8 or less, or 8.0 or less. By being present in the sealed system, the pH of the culture medium contained in the sealed system tends to be kept within the above range while the cells are cultured. When the pH of the culture medium contained in the sealed system is 7.0 or more or 8.0 or more, lactic acid is neutralized in the sealed system during culture to suppress a decrease in pH, which is preferable.

During culture of the cells in the sealed system, at least one or all of carbon dioxide gas, nitrogen gas, and oxygen gas may not be supplied into the sealed system. Further, during culture of the cells in the sealed system, it is not necessary to control the carbon dioxide concentration in the sealed system. During culture of the cells in the sealed system, the carbon dioxide concentration outside the sealed system need not be controlled. For example, the sealed system may not be placed in a CO₂ incubator. However, it is not precluded to place the sealed system in a CO₂ incubator.

When culturing the cells in the sealed system, it is preferable that there is no or less gas layer such as an air layer in the sealed system. Therefore, it is preferable that the culture medium is filled in the sealed system so that no or less gas layers remain in the sealed system.

During culture of the cells in the sealed system, the culture medium may be circulated in the sealed system so as not to touch the outside air. In the sealed system, a semipermeable membrane may be placed between the cell suspension and the circulating culture medium, and the active ingredient of the culture medium may be permeated into the cell suspension via the semipermeable membrane.

Within the sealed system, the cells are cultured, for example, for 3 hours or more, 1 day or more, 14 days or more, or 30 days or more. However, the sealed system may be opened for passage, replacement of culture medium, and addition of culture medium.

It should be noted that there is no need to replace or add culture medium between seeding and passage of cells. Between seeding and passage, the cells in the sealed system are cultured while the sealed system is never opened. For example, between seeding and passage, the cells in the sealed system are cultured while the sealed system is never opened for 1 day or more, 5 days or more, or 10 days or more.

The culture medium need not be exchanged and added between passages of the cells. Between passages, the cells in the sealed system are cultured while the sealed system is never opened. For example, between passages, the cells in the sealed system are cultured while the sealed system is never opened for 1 day or more, 5 days or more, or 10 days or more.

Alternatively, the sealed system may be opened and the culture medium may be added or replaced between seeding and passage of the cells. Further, between passages of the cells, the sealed system may be opened and culture medium may be added or replaced. The culture medium may be added or replaced every 1 day or more, every 2 days or more, or every 5 days or more. The cells in the sealed system are cultured while the sealed system is never opened except for addition or replacement of the culture medium and passage.

Further, induction method of cells according to an embodiment includes inducing cells in the sealed system. The sealed system is as described above. Induction refers to reprogramming; initialization; differentiation transformation (Transdifferentiation or Lineage reprogramming); differentiation induction; cell fate reprogramming (Cell fate reprogramming); and the like.

The cells induced in the sealed system may be cells introduced beforehand with inducing factor outside the sealed system. Alternatively, an inducing factor may be added to the culture medium in the sealed system, and the inducing factor may be introduced into cells cultured in the sealed system which have not been introduced with the inducing factor to induce the cells in the sealed system.

The cells induced in the sealed system may be animal cells or plant cells.

The cells may be induced in a liquid medium in the sealed system or may be induced in a gel culture medium in the sealed system. Further, the cells may be induced while being subjected to adherent culture in the sealed system or may be induced while being subjected to floating culture. During induction of the cells in the sealed system, the culture medium may or may not be agitated. When inducing cells while being subjected to adherent culture, a feeder cell may be used, or a feeder cell may not be used. Feeder cells may not be used when inducing cells while being subjected to floating culture.

The cells within the sealed system may be induced into stem cells, such as iPS cells. The cells within the sealed system may be derived to other types of cells than stem cells. Stem cells, such as iPS cells and ES cells, may be induced into other types of cells within the sealed system.

The cells induced to iPS cells in the sealed system may be blood cells, such as hemocytes. Alternatively, the cells induced in the sealed system into iPS cells may be fibroblasts, medullary stem cells, keratinocytes, hair papilla cells, oral epithelial cells, somatic stem progenitor cells, and the like. The cells in the sealed system may be induced, for example, into blood cells, nerve cells, cardiac muscle system cells, epithelial cells, mesenchymal cells, hepatocytes, insulin producing cells, retinal pigment epithelial cells, and corneal cells.

The hemocytes are separated from the blood. Blood is, for example, but not limited to, peripheral blood and umbilical cord blood. Blood may be collected from an adult or from an underage person. When collecting blood, anticoagulants such as ethylenediaminetetraacetic acid (EDTA), heparin, and liquid A (ACD-A) in the reference blood-preserving solution for biological products are used.

The hemocytes are, for example, nucleated cells, such as monocytes (mononuclear cells), neutrophils, eosinophils, lymphocytes, macrophages, blood stem/progenitor cells, and vascular endothelial cells, and do not contain red blood cells and platelets. The hemocytes may be, for example, vascular endothelial progenitor cells, blood stem/progenitor cells, T cells, or B cells. The T cells are, for example, αβT cells.

Monocytes are separated from blood using a medium for separating the hemocytes, and a centrifuge device or the like. When Ficoll is used as a medium for separating hemocytes, a method for separating monocytes is as follows.

Since the separation accuracy of monocytes tends to be poor at low temperature, the centrifuge is set at a range of from 4 °C to 42 °C, and preferably at 18 °C. 10 µL to 50 mL of blood is collected from an adult or underage person, chelating agents containing EDTA are added, and gently mixed so that the blood does not solidify. Further, 5 mL of the medium for separating human lymphocytes (Ficoll-Paque PREMIUM, GE Healthcare Japan) are dispensed into two 15 mL tubes. 5 mL of PBS is added to 5 mL of blood and diluted, and the mixture is layered on top of the medium for human lymphocyte separation in the tube at 5 mL portions. At this time, the diluted blood is slowly added onto the medium by passing through the tube wall of the tube so as not to disturb the interface.

The solution in the tube is centrifuged at 10×g to 1000×g, preferably 400×g, at from 4 °C to 42 °C, preferably 18 °C, for 5 minutes to 2 hours, preferably 30 minutes. After centrifugation, a white, cloudy interlayer appears in the tube. This white, cloudy interlayer contains monocytes. The white, cloudy interlayer in the tube is slowly collected with a pipetman and transferred to a new 15 mL tube. At this time, the lower layer should not be sucked off. The white and cloudy intermediate layer can be recovered by about 1 mL from one tube. The intermediate layers for two tubes are transferred together into one tube.

To the recovered monocytes, 1 mL to 48 mL, preferably 12 mL of PBS is added, and the solution is further centrifuged at from 10×g to 1000×g, preferably at 200×g, at 4 °C to 42 °C, preferably from 1 minutes to 60 minutes, preferably 10 minutes at 18 °C. Thereafter, the supernatant of the solution is removed by aspiration using an aspirator, and 1 mL to 12 mL, preferably 3 mL of a known composition serum-free hematopoietic cell culture medium (X-VIVO(R) 10, Lonza) is added and suspended to obtain a monocyte suspension. Of the suspension, 10 µL of monocyte suspension is stained with trypan blue and counted on a hemocytometer.

When vacutaina(R) (BD) is used as a blood collection tube, a method of separating monocytes is as follows.

Since the separation accuracy of monocytes tends to be poor at low temperature, the centrifuge is set at a range of from 4 °C to 42 °C, and preferably at 18 °C. 8 ml of blood was collected from an adult or an underage person using vascular collection (vacuteina(R), BD), and mixed with the anticoagulant by mixing by inverting. Thereafter, the balance is adjusted and the solution is centrifuged at from 4 °C to 42 °C, preferably at 18 °C, at from 100×g to 3000×g, preferably at from 1500×g to 1800×g, for 1 minutes to 60 minutes, preferably 20 minutes in a swing rotor. After centrifugation, the upper layer, which is the plasma layer, is removed and pipetted to suspend the monocyte layer and the blood cells sticking to the gel to obtain a suspension. The resulting suspension is transferred to another 15 mL tube.

To the suspension of the 15 mL tube 1 mL to 14 mL, preferably 12 mL of PBS is added, and the suspension is centrifuged at from 4 °C to 42 °C, preferably at 18 °C, at from 100×g to 3000×g,preferably at 200×g, for 1 minutes to 60 minutes, preferably 5 minutes. After centrifugation, the supernatant is removed with an aspirator. Further, the hemolytic agent (PharmLyse(R), 10 times concentration, BD) is diluted to 1 fold concentration with sterile water. The pellet in the 15 mL tube is loosened by tapping, and 1 mL to 14 mL, preferably 1 mL of hemolysate is added. Thereafter, the solution is allowed to stand for 1 minute to 60 minutes, preferably 1 minute while being protected from light at room temperature.

Next, 1 mL to 14 mL, preferably 12 mL of PBS is added to the 15 mL tube and centrifuged for 5 minutes, at from 4 °C to 42 °C, preferably at room temperature, at from 100×g to 3000×g, preferably at 200×g, for 1 minute to 60 minutes. After centrifugation, the supernatant is removed with an aspirator, and 1 mL to 15 mL, preferably 3 mL of known composition serum-free hematopoietic cells culture medium (X-VIVO(R) 10, Lonza) is added and suspended to obtain a monocyte suspension. Of the suspension, 10 µL of monocyte suspension is stained with trypan blue and counted on a hemocytometer.

The method of separating monocytes from blood is not limited to the method described above, and for example, a dialysis membrane may be used to separate monocytes from blood. Further, filters such as pure cell select system for concentrating whole blood mononuclear cells(R), PALL), a purifier for removing blood cells (cell sorber E(R), Asahi Kasei), and a leukocyte removing filter for platelet preparation (Sepacell PL(R), PLX-5B-SCD, Asahi Kasei) can also be used.

Monocytes may be separated using an erythrocyte sedimentation agent capable of separating nucleated cells by gravity sedimentation or centrifugation of erythrocytes. Examples of the erythrocyte sedimentation agent include HetaSep(R) (STEMCELL Technologies) and HES40(NIPRO).

Further, as monocytes, CTL-UP1 sold by Cellular Technology Limited Co., PBMC-001 of Sanguine Biosciences Co., Ltd., or the like may be used.

Alternatively, the hemocytes may be used by thawing hemocytes cryopreserved using cell cryopreservation solutions such as Cell Banker 1, Stem Cell Banker GMP grade, and Stem Cell Banker DMSO free GMP grade (Zenoac).

In thawing monocytes, first, 1 mL to 15 mL, preferably 8 mL of known composition serum-free hematopoietic cells culture medium (X-VIVO(R) 10, Lonza) is placed in a 15 mL tube, and a tube containing frozen mononuclear cells is placed in a warm bath at 4 °C to 42 °C, preferably 37 °C, to start lysing the mononuclear cells. Thereafter, with a little ice remaining, the tube containing monocytes is pulled up from the warm bath, and the monocytes are transferred to a tube containing known composition serum-free hematopoietic cell culture medium. Of this, 10 µL of monocyte suspension is stained with trypan blue and counted on a hemocytometer.

The hemocytes may be separated based on cell surface markers. Blood stem/progenitor cells are positive for CD34. T cells are positive for any of CD3, CD4, CD8. B cells are positive for any of CD10, CD19, CD20. Blood stem/progenitor cells, T cells, or B cells are separated from hemocytes using, for example, an automated magnetic cell separator and immunomagnetic bead. Alternatively, pre-separated monocytes may be prepared. However, hemocytes which have not been separated based on cell surface markers may be used.

CD34 positive cells are stem/progenitor cells and tend to be susceptible to reprogramming. When iPS cells are produced using T cells which are CD3 positive cells, iPS cells derived from T cells retain the type of TCR-combination, and thus tend to be able to efficiently differentiated and induced to T cells.

An inducing factor is introduced into the cells under adherent culture. Alternatively, the inducing factor is introduced into cells under floating culture with gel culture medium. The inducing factor may be an RNA. The inducing factor may be included in Sendai virus. Alternatively, the inducing factor may be introduced into cells by transfection. The inducing factor may be a DNA. The inducing factor may be included in plasmids.

The inducing factor may be included, for example, in adenoviruses, lentiviruses, and retroviruses.

The inducing factor may be proteins.

As Sendai virus, a CytoTune(R) (Invitrogen) can be used. An indicator of titer of Sendai virus is multiplicity of infection (MOI). The MOI of Sendai virus is, for example, 0.1 to 100.0, or 1.0 to 50.0.

When inducing cells into stem cells, for example, the inducing factor introduced into the stem cells comprises mRNA of OCT3/4, mRNA of SOX2, mRNA of KLF4, and mRNA of c-MYC. As inducing factor, M₃O which is an improved OCT4 may be used. The inducing factor may further comprise a mRNA of at least one agent selected from the group consisting of LIN28A, FOXH1, LIN28B, GLIS1, p53-dominant negative, p53-P275S, L-MYC, NANOG, DPPA2, DPPA4, DPPA5, ZIC3, BCL-2, E-RAS, TPT1, SALL2, NAC1, DAX1, TERT, ZNF206, FOXD3, REX1, UTF1, KLF2, KLF5, ESRRB, miR-291-3p, miR-294, miR-295, NR5A1, NR5A2, TBX3, MBD3sh, TH2A, TH2B and P53DD. These mRNA are available from TriLink.

The mRNAs contained in the inducing factor may be modified with at least one selected from the group consisting of Pseudouridine (ψ), 5-methylcytosine (m⁵C), 5-methyluridine (5meU or m⁵U), N1-methylpseudouridine (me1Ψ), 5-methoxyuridine (5moU), 5-hydroxymethyluridine (5hmU), 5-formyluridine (5fU), 5-carboxymethylesteruridine (5camU), thienoguanosine (thG), N4-methylcytidine (me⁴C), 5-methylcytidine (me⁵C), 5-methyloxycytisine (5moC), 5-hydroxymethylcytidine (5hmC), 5-hydroxycytidine (5hoC), 5-formcytidine (5fC), 5-carboxycytidine (5caC), N⁶-methyl-2-aminoadenosine (m⁶DAP), diaminopurine (DAP), 2'-O-methyluridine (Um or m^{2'-O}U), 2-thiouridine (s²U), N⁶-methyladenosine (m⁶A).

Cytosine may be substituted with 5-methylcytosine (m⁵C). Uracil may be substituted with pseudouracil.

mRNA contained in the inducing factor may be polyadenylated.

mRNA contained in the inducing factor may be prepared by polyadenylation of in vitro transcribed (IVT) RNAs. mRNA may be polyadenylated during IVTs by using DNA templates encoding poly(A) termini. mRNA may be capped. In order to maximize the efficiencies of expression in the cells, it is preferred that most mRNA molecules contain caps. mRNA may have a 5'cap[m7G(5')ppp(5')G] configuration. The sequence stabilizes mRNA and promotes transcription. 5'triphosphate may be removed from mRNA having 5'triphosphate by dephosphorylation. mRNA may have[3'O-Me-m7G(5')ppp(5')G] as Anti-Reverse Cap Analog(ARCA). ARCA is a sequence inserted prior to the transcription initiation point, and mRNA transcribed is twice as efficient. mRNA may have a PolyA tail.

mRNA contained in the inducing factor may have been treated with ribonuclease III (RNaseIII).

mRNA contained in inducing factor may be a replicative RNA capable of self-proliferation. Replicative RNAs are self-replicating RNAs that, unlike ordinary RNAs, also have the ability to express the necessary proteins to replicate RNAs. Replicative RNAs are derived from Venezuelan horse encephalite (VEE) viruses, a kind of alpha viruses. Transfection of replicative RNA into cells allows the cells to express RNA that continues to make reprogramming factors, thus eliminating the need to introduce inducing factor RNA a plurality of times into the cells.

The sequence of the replicative RNA may comprise a sequence obtained from an alphavirus selected from the group consisting of alphavirus replicon RNA, Eastern equine encephalitis virus (EEE), Venezuelan equine encephalitis virus (VEE), Everglades virus, Mucambo virus, Pixuna virus, and Western equine encephalitis virus (WEE).

Further, the replicative RNA may include a sequence obtained from alphavirus selected from the group consisting of a Sindbis virus, a Semliki Forest virus, a Middelburg virus, a Chikungunya virus, a O'nyong-nyong virus, a Ross River virus, a Barmah Forest virus, a Getah virus, a Sagiyama virus, a Bebaru virus, a Mayaro virus, a Una virus, an Aura virus, a Whataroa virus, a Babanki virus, a Kyzylagach virus, a Highlands J virus, a Fort Morgan virus, a Ndumu virus, and a Buggy Creek virus.

Replicative RNAs include, for example, (VEE RNA replicase)-(promoter)-(RF1)-(self-cleaving peptide)-(RF2)-(self-cleaving peptide)-(RF3)-(IRES or core promoter)-(RF4)-(IRES or optional promoter)-(optional marker)-(VEE 3'UTR and polyA tail)-(optional marker)-promoters, from 5' to 3'. The above RF1-4 are factors that induce cell dedifferentiate into pluripotent cells differentiation. The above RF2-3, RF3-4, RF4 are optional. RF1-4 may be selected from the group consisting of OCT-4, KLF4, SOX-2, c-MYC, LIN28A, LIN28B, GLIS1, FOXH1, p53-dominant negative, p53-P275S, L-MYC, NANOG, DPPA2, DPPA4, DPPA5, ZIC3, BCL-2, E-RAS, TPT1, SALL2, NAC1, DAX1, TERT, ZNF206, FOXD3, REX1, UTF1, KLF2, KLF5, ESRRB, miR-291-3p, miR-294, miR-295, NR5A1, NR5A2, TBX3, MBD3sh, TH2A and TH2B.

The culture medium in which the cells into which the inducing factor is introduced is cultured is appropriately selected according to the type of the cells into which the inducing factor is introduced. Further, the culture medium in which the cells into which the inducing factor has been introduced is cultured is appropriately selected according to the type of the cells into which the inducing factor has been introduced.

As described above, the culture medium may not contain, for example, growth factor such as bFGF, or may contain growth factor at a low concentration. Further, the culture medium may contain no tgf-β or may contain tgf-β at a low concentration. The culture medium may include at least one material selected from the group consisting of cadherin, laminin, fibronectin, and vitronectin.

When the culture medium is a gel culture medium, the culture medium may contain at least 1 kind of polymer compounds as described above. Further, the gel culture medium may contain methylcellulose. Alternatively, the gel culture medium may include a temperature-sensitive gel, as described above.

During induction of the cells in the sealed system, the temperature of the culture medium in the sealed system is similar to, for example, the temperature during culture of the cells in the sealed system described above.

The pH of the culture medium placed in the sealed system where the cells are induced is similar, for example, to the pH of the culture medium placed in the sealed system where the cells are cultured as described above.

During induction of the cells in the sealed system, at least one or all of carbon dioxide gas, nitrogen gas, and oxygen gas may not be supplied into the sealed system. Further, during induction of the cells in the sealed system, it is not necessary to control the carbon dioxide concentration in the sealed system. During induction of the cells in the sealed system, the carbon dioxide concentration outside the sealed system need not be controlled. For example, the sealed system may not be placed in a CO₂ incubator. However, it is not precluded to place the sealed system in a CO₂ incubator.

When inducing the cells in the sealed system, it is preferable that there is no or less gas layer such as an air layer in the sealed system. Therefore, it is preferable that the culture medium is filled in the sealed system so that no or less gas layers remain in the sealed system.

During induction of the cells in the sealed system, the culture medium may be circulated in the sealed system so as not to touch the outside air. In the sealed system, a semipermeable membrane may be placed between the cell suspension and the circulating culture medium, and the active ingredient of the culture medium may be permeated into the cell suspension via the semipermeable membrane.

Within the sealed system, the cells are induced while being cultured, for example, for 1 day or more, 14 days or more, or 30 days or more. However, the sealed system may be opened for passage, replacement of culture medium, and addition of culture medium.

It should be noted that there is no need to replace or add culture medium between seeding and passage of cells. Between seeding and passaging, the cells in the sealed system are induced while being cultured while the sealed system is never opened. For example, between seeding and passage, the cells in the sealed system are induced while being cultured while the sealed system is never opened for 1 day or more, 5 days or more, or 10 days or more.

The culture medium need not be exchanged and added between passages of the cells. Between passages, the cells in the sealed system are induced while being cultured while the sealed system is never opened. For example, between passages, the cells in the sealed system are induced while being cultured while the sealed system is never opened for 1 day or more, 5 days or more, or 10 days or more.

Alternatively, the sealed system may be opened and the culture medium may be added or replaced between seeding and passage of the cells. Further, between passages of the cells, the sealed system may be opened and culture medium may be added or replaced. The culture medium may be added or replaced every 1 day or more, every 2 days or more, or every 5 days or more. The cells in the sealed system are induced while being cultured while the sealed system is never opened except for addition or replacement of the culture medium and passage.

Whether the cell into which the inducing factor has been introduced has been induced (reprogrammed) to iPS cell can be confirmed, for example, from the morphology of the cells. Alternatively, whether the cell has been induced into an iPS cell can be determined by analyzing with a cytoflowmeter, whether at least one surface marker selected from: TRA-1-60, TRA-1-81, SSEA-1, and SSEA5 which are cell surface markers indicative of undifferentiation, is positive or not. TRA-1-60 is an antigen specific for iPS/ES cells and is not detected in differentiated cells. Since iPS cells can only be obtained from TRA-1-60 positive fraction, TRA-1-60 positive cells are considered to be species of iPS cells.

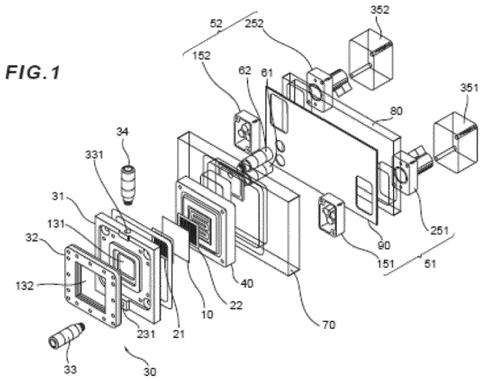

The sealed system for culturing or inducing cells inside according to an embodiment may include, for example, a cell culture vessel as shown in Fig.1. The cell culture vessel comprises a culture component permeable member 10 through which culture component is permeable, a culture chamber 30 for culturing cells covering one surface of the culture component permeable member 10 and holding a culture medium containing cells, and a culture medium holding chamber 40 for holding culture medium covering the other surface of the culture component permeable member 10. The culture medium containing cells in the culture chamber 30 is accessible to the culture component permeable member 10. Further, the culture medium in the culture medium holding chamber 40 is accessible to the culture component permeable member 10. The culture medium within the culture medium holding chamber 40 contains no cells.

The culture component permeable member 10 permeates the active ingredient of the culture medium in the culture medium holding chamber 40 into the culture medium containing cells in the culture chamber 30. Further, the culture component permeable member 10 may allow waste products in the culture medium containing cells in the culture chamber 30 to permeate into the culture medium in the culture medium holding chamber 40. As culture component permeable member 10, for example, semipermeable membrane and mesh can be used. Semipermeable membrane includes a dialysis membrane.

When the culture component permeable member 10 is a semipermeable membrane, the fractional molecular weight of semipermeable membrane is, for example, greater than or equal to 0.1KDa, greater than or equal to 10KDa, or greater than or equal to 50KDa. The semipermeable membrane consists of, for example, cellulose esters, ethyl cellulose, cellulose esters, regenerated cellulose, polysulfone, polyacrylonitrile, polymethyl methacrylate, ethylene vinyl alcohol copolymers, polyester-based polymer alloys, polycarbonates, polyamides, cellulose acetates, cellulose diacetates, cellulose triacetates, copper ammonium rayon, saponified cellulose, hemophane membranes, phosphatidylcholine membranes, and vitamin E-coated membranes.

When the culture component permeable member 10 is a mesh, the mesh has pores smaller than the cells or cell masses to be cultured in the culture chamber 30. This prevents cells or cell masses in the culture chamber 30 from migrating into the culture medium holding chamber 40. The material of the mesh is, for example, a resin and a metal, but is not particularly limited. The surface of the culture component permeable member 10 may be non-adherent to cells.

The cell culture vessel according to the embodiment may further include a culture side plate 21 and a culture medium side plate 22 each provided with an opening sandwiching the culture component permeable member 10. The culture side plate 21 and the culture medium side plate 22 hold the culture component permeable member 10 so as to sandwich the culture component permeable member 10 so as to suppress the culture component permeable member 10 from being fluctuated by the pressure of the culture medium containing cells in the culture chamber 30 and the pressure of the culture medium holding chamber 40. Thus, the pressure variation prevents the culture component permeable member 10 from contacting the inner wall of the culture chamber 30 or the culture medium holding chamber 40. The culture side plate 21 and the culture medium side plate 22 have a hardness that is not displaced by the pressure received from the culture medium containing cells in the culture chamber 30 and the culture medium in the culture medium holding chamber 40. The material of the culture side plate 21 and the culture medium side plate 22 is, for example, a resin and a metal, but is not particularly limited. The surface of the culture side plate 21 may be non-adherent to cells. It is to be noted that, when the culture component permeable member 10 is not displaced due to the pressure received from the culture medium containing cells in the culture chamber 30 and the culture medium in the culture medium holding chamber 40, the culture side plate 21 and the culture medium side plate 22 may be omitted.

The culture side plate 21 is provided with openings so that the culture medium containing cells in the culture chamber 30 can be brought into contact with the culture component permeable member 10. Further, openings are provided in the culture medium side plate 22 so that the culture medium in the culture medium holding chamber 40 can be brought into contact with the culture component permeable member 10. Through the openings of the culture side plate 21, the component of the culture medium containing cells in the culture chamber 30 and the component of the culture medium in the culture medium holding chamber 40 can permeate through the culture component permeable member 10. The shapes of the openings provided in each of the culture side plate 21 and the culture medium side plate 22 are, for example, circles, but are not particularly limited. The openings provided in each of the culture side plate 21 and the culture medium side plate 22 have a size within a range in which the culture component permeable member 10 can be suppressed from being displaced. The openings are provided in each of the culture side plate 21 and the culture medium side plate 22, for example, in a grid pattern or randomly.

The culture side plate 21 may have a dark color such as black, for example. When the culture side plate 21 has a dark color, cells in the culture medium containing cells can be visually recognized or imaged with high contrast using the culture side plate 21 as a background. If the size of the area or the like of the portion where the openings of the culture side plate 21 is not provided is larger than that of the cells or the cell masses, it becomes easy to visualize or image the cells or the cell masses with high contrast with the portion where the openings of the culture side plate 21 are not provided as the background. However, by adjusting the light to be irradiated on the cells or cell masses, even if the culture component permeable member 10 and the culture side plate 21 are transparent, the cells or cell masses can be visually recognized or imaged.

The culture chamber 30 and the culture medium holding chamber 40 may be fixed by screws, pins, electromagnets, or the like. The contact portion of the culture chamber 30 and at least a portion of one surface of the culture side plate 21 are brought into close contact with each other. At least a portion of the other surface of the culture side plate 21 and at least a portion of one surface of the culture component permeable member 10 are brought into close contact with each other. At least a portion of the other surface of the culture component permeable member 10 and at least a portion of one surface of the culture medium side plate 22 are brought into close contact with each other. At least a portion of the other surface of the culture medium side plate 22 and a contact portion of the culture medium holding chamber 40 are brought into close contact with each other. A packing or the like may be used as appropriate for the close contacts. The packing may be disposed between, for example, the culture component permeable member 10 and the culture chamber 30. The packing may be disposed between the periphery of the culture component permeable member 10 and the culture chamber 30. The outer diameter of the packing disposed between the culture component permeable member 10 and the culture chamber 30 may be larger than the outer diameter of the culture component permeable member 10. Further, a packing may be disposed between the culture component permeable member 10 and the culture medium holding chamber 40, for example. The packing may be placed between the periphery of the culture component permeable member 10 and the culture medium holding chamber 40. The outer diameter of the packing disposed between the culture component permeable member 10 and the culture medium holding chamber 40 may be larger than the outer diameter of the culture component permeable member 10.

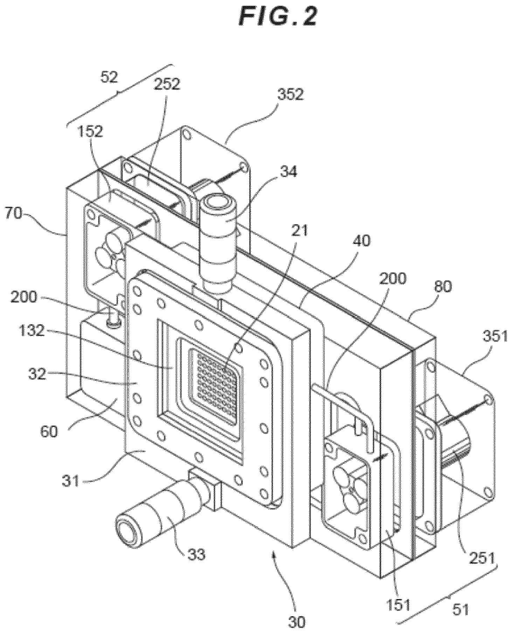

The culture chamber 30 includes, for example, a cover 32 covering a housing 31 and a housing 31. The housing 31 and the covering 32 may be integrated. The inner wall of the culture chamber 30 may be coated with a cell non-adhesive material such as poly-HEMA(poly 2-hydroxyethyl methacrylate) to render the inner wall of the culture chamber 30 cell non-adhesive so that cells do not adhere thereto. The housing 31 is provided with an opening 131 for exposing the culture component permeable member 10 through an opening in the culture side plate 21. As shown in Fig. 2, the cover 32 of the culture chamber 30 is provided with a window 132 capable of observing the culture medium containing cells in the culture chamber 30. As a material of the window 132, for example, glass and resin can be used.

The cell culture vessel according to the embodiment may include a temperature controller for heating and cooling the windows 132. The temperature controller may be a transparent heater, such as a transparent conductive film, disposed in the window 132 and heating the window. Alternatively, the cell culture vessel according to the embodiment may include a temperature controller for heating and cooling the housing 31 or the cover 32 of the culture chamber 30. By adjusting the temperature of any of the housing 31, the covering 32, and the windows 132 by the temperature controller, it is possible to adjust the temperature of the culture medium containing cells in culture chamber 30. The cell culture vessel according to the embodiment may further include a thermometer for measuring the temperature of the culture medium containing cells in the culture chamber 30. The thermometer may measure the temperature of the culture medium containing cells based on the temperature of the culture chamber 30 without contacting the culture medium containing cells, or may directly measure the temperature of the culture medium containing cells by contacting the culture medium containing cells. In this case, the temperature controller may be feedback-controlled so that the temperature of the culture medium containing cells becomes a predetermined temperature.

As shown in Fig.1, the culture chamber 30 is provided with a feed port 231 for supplying fluid into the culture chamber 30 and a discharge port 331 for discharging fluid in the culture chamber 30. For example, a plug 33 shown in Fig.2, is inserted into the feed port 231 to which a feeder, such as bags, bellows and syringes for supplying fluids, can be connected. The feeder may be a fluid machine such as a pump. However, an infusion device may be directly connected to the feed port 231 shown in Fig.1. The feeder is detachable from the feed port 231, and when the feeder is not connected to the feed port 231, the feed port 231 is sealable and no fluid exchange occurs between the culture chamber 30 and the outside through the feed port 231.

The plug 33 may be a needleless connector. The needleless connector may be split-septum type or mechanical valve type. When the plug 33 is a split-septum type needleless connector, the plug 33 comprises a slitted disk valve. When supplying fluids into the culture chamber 30, a feeder or a flow path connected to the feeder is inserted into the slits of the disc valve. When the feeder or the flow path connected to the feeder is not inserted into the slits, the slits are sealed. When the feeder or the flow path connected to the feeder is inserted into the slits, the disc valve fits closely against the periphery of the flow path connected to the feeder or the flow path connected to the feeder. Therefore, even when the feeder or the flow path connected to the feeder is inserted into the plug 33, the outside air does not enter the culture chamber 30 through the plug 33. However, the plug 33 may be a connector into which a needle is inserted.

Further, inserted into the discharge port 331 is a plug 34 shown in Fig.2, to which a discharger, such as bags, bellows and syringes, for draining fluids in the culture chamber 30 can be connected. The discharger may be fluid machines such as pumps. However, the discharger may be directly connected to the discharge port 331 shown in Fig.1. The discharger may actively aspirate fluid in the culture chamber 30. Alternatively, the discharger may passively increase the internal volume in response to the pressure in the culture chamber 30 and receive the fluid extruded from the culture chamber 30. When the discharger is detachable from the discharge port 331 and not connected to the discharge port 331, the discharge port 331 is sealable, and there is no changeover of fluids in or out of the culture chamber 30 via the discharge port 331. The plug 34 may be a needleless connector. The needleless connector may be split-septum type or mechanical valve type. Even when the discharger or the flow path connected to the discharger is inserted into the plug 34, the outside air does not enter the culture chamber 30 through the plug 34. However, the plug 34 may be a connector into which a needle is inserted.

For example, when culture chamber 30 is in close contact with the culture medium holding chamber 40 with the culture side plate 21, the culture component permeable member 10, and the culture medium side plate 22 interposed therebetween, and air is contained in culture chamber 30, the culture medium containing cells can be placed in the culture chamber 30 shown in Fig.2 by injecting the culture medium containing cells from the feed port 231 into the culture chamber 30 while exhausting the air in the culture chamber 30 from the discharge port 331. It is also possible to completely eliminate an air layer in the culture chamber 30. However, an air layer may be remained in the culture chamber 30. When the culture medium containing cells is already contained in the culture chamber 30, at least a portion of the culture medium containing cells in the culture chamber 30 shown in Fig.2 can be replaced by injecting other culture medium containing cells from the feed port 231 into the culture chamber 30 while discharging the culture medium containing cells in the culture chamber 30 from the discharge port 331 shown in Fig.1.

The cell culture vessel according to the embodiment may further include a culture chamber holding member capable of holding the culture chamber 30 and capable of adjusting the slope of the culture chamber 30. By adjusting the slope of the culture chamber 30, it is easy to discharge gases such as air in the culture chamber 30.

The feed port 231 and the discharge port 331 of the culture chamber 30 can be closed by a plug or the like. Alternatively, plugs 33 and plugs 34 connected to the feed port 231 and the discharge port 331 of the culture chamber 30, respectively, can close them. Alternatively, the feed port 231 of the culture chamber 30 can be shielded from the outside by being connected to the feeder, and the discharge port 331 of the culture chamber 30 can be shielded from the outside by being connected to the discharger. When the feed port 231 and the discharge port 331 are closed and the culture chamber 30 is brought into close contact with the culture medium holding chamber 40 as shown in Fig.2, the inside of the culture chamber 30 is sealed from the air outside the culture chamber 30. Thus, entry of the outside air into the culture chamber 30 is suppressed, and change in the pH of the culture medium containing cells in the culture chamber 30 is suppressed and kept within a predetermined range. It is to be noted that, according to the findings of the present inventors, since the cells can be cultured in a completely closed sealed space, it is not necessary to actively supply carbon dioxide gas, nitrogen gas, oxygen gas, and the like into culture chamber 30. Therefore, the culture chamber 30 does not have to be placed in a CO₂ incubator. Further, since cells, microorganisms, viruses, dust, and the like existing outside the culture chamber 30 do not enter into the sealed culture chamber 30, the cleanliness in the culture chamber 30 is maintained. Therefore, it is not necessary to place the culture chamber 30 in a clean room. The culture chamber 30 may be embedded within a gas impermeable material. In other words, the culture chamber 30 may be embedded in the gas impermeable material.

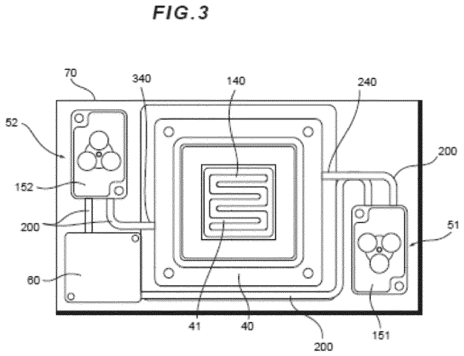

The culture medium holding chamber 40 shown in Fig.1 is provided with an opening 140 shown in Fig.3 for exposing the culture component permeable member 10 through the opening of the culture medium side-plate 22. The opening 140 is covered with the culture component permeable member 10 shown in Fig.1. Further, the culture medium holding chamber 40 shown in Fig.3 is provided with an inlet 240 for introducing fluid into the culture medium holding chamber 40 and a discharge port 340 for discharging fluid in the culture medium holding chamber 40. Further, a plurality of rectifying plates 41 may be disposed in the culture medium holding chamber 40. The plurality of rectifying plates 41 are arranged so as to project alternately from, for example, the opposing inner walls of the culture medium holding chamber 40.

For example, when the culture medium holding chamber 40 is in close contact with the culture chamber 30 with the culture medium side plate 22, the culture component permeable member 10, and the culture side plate 21 shown in Fig.1 interposed therebetween, and air is contained in the culture medium holding chamber 40, the cell culture medium can be introduced into the culture medium holding chamber 40 by injecting the cell culture medium into the culture medium holding chamber 40 through the inlet 240 while exhausting the air in the culture medium holding chamber 40 from the discharge port 340 shown in Fig.3. In addition, when the culture medium is already contained in the culture medium holding chamber 40, it is possible to flow the cell culture medium into the culture medium holding chamber 40 by injecting the cell culture medium into the culture medium holding chamber 40 from the inlet 240 while discharging the cell culture medium in the culture medium holding chamber 40 from the discharge port 340.

When a plurality of rectifying plates 41 are arranged in the culture medium holding chamber 40, the culture medium flows along the plurality of rectifying plates 41 from the inlet 240 towards the discharge port 340 in culture medium holding chamber 40. Therefore, there is a chance that the components of the culture medium is contacted with the culture component permeable member 10.

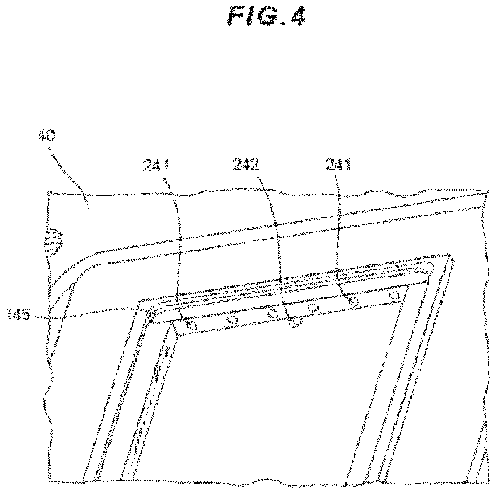

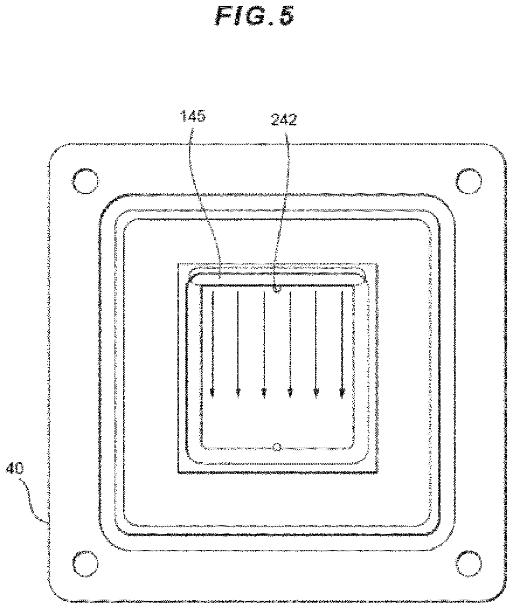

Alternatively, as shown in Fig.4, one or a plurality of discharge ports 241 communicating with the inlet port 240 shown in Fig.3 may be provided on the inner wall of the culture medium holding chamber 40. The plurality of discharge ports 241 shown in Fig.4 are provided, for example, in a row. The number and arrangement of the plurality of discharge ports 241 may be uniformly arranged or may be randomly arranged. The number of the plurality of discharge ports 241 and the arrangement are set according to the characteristics such as viscosity of culture medium. As shown in Fig.5, by discharging the culture medium from the plurality of discharge ports 241, it is possible to improve the uniformity of the culture medium contacting the culture component permeable member 10 in the culture medium holding chamber 40.

A discharge block 145 provided with one or a plurality of discharge ports 241 may be inserted in the inner wall of the culture medium holding chamber 40. For example, a discharge block 145 having different patterns such as the number and arrangement of the plurality of discharge ports 241 may be prepared, and may be selectively used according to the characteristics of the culture medium and the cells to be cultured. The upper side of the inner wall of the culture medium holding chamber 40 may be bent or curved upwardly or downwardly with respect to the gravitational force. The under side of the inner wall of the culture medium holding chamber 40 may be bent or curved upwardly or downwardly with respect to the gravitational force.

As shown in Fig.4 and Fig.5, an opening 242 may be provided in the vicinity of the plurality of discharge ports 241 on the inner wall of the culture medium holding chamber 40. As the culture medium discharged from the plurality of discharge ports 241 accumulates in the culture medium holding chamber 40, the air in the culture medium holding chamber 40 flows out through the opening 242. After placing the culture medium into the culture medium holding chamber 40, the opening 242 may be sealed.

As shown in Fig.3, the inlet 240 and the discharge port 340 of the culture medium holding chamber 40 are connected by a culture medium channel 200, and the culture medium may be circulated though the culture medium holding chamber 40 and the culture medium channel 200. The culture medium channel 200 may include a resin tube, a silicon tube, or the like. The culture medium channel 200 may be embedded within a gas impermeable material. In other words, the culture medium channel 200 may be embedded in the gas impermeable material. For example, the culture medium channel 200 may be a hole provided in a member made of resin, glass, metal, or the like. In that case, for example, the culture medium channel 200 is formed by attaching the members provided with concave portions to each other. The culture medium channel 200 may be provided with a fluid machine for introducing the culture medium into the culture medium holding chamber 40 and discharging the culture medium from within the culture medium holding chamber 40. The fluid machine includes, for example, an introduction fluid machine 51 for introducing culture medium into the culture medium holding chamber 40, and a discharge fluid machine 52 for discharging culture medium from within the culture medium holding chamber 40.

As the introduction fluid machine 51 and the discharge fluid machine 52 shown in Fig.1, a positive displacement pump can be used. Examples of positive displacement pumps include reciprocating pumps including piston pumps, plunger pumps, and diaphragm pumps, or rotary pumps including gear pumps, vane pumps, and screw pumps. Examples of diaphragm pumps include tubing pumps and piezoelectric (piezo) pumps. The tubing pump may also be referred to as a peristaltic pump. Further, microfluidic chip modules combining various types of pumps may also be used.

Sealed pumps, such as peristaltic pumps(R), tubing pumps, and diaphragm pumps, can be used to deliver liquid without direct contact of the pump to the culture medium within the culture medium channel 200 shown in Fig.3. Alternatively, a syringe pump may be used as the introduction fluid machine 51 and the discharge fluid machine 52. A pump other than a sealed pump may also be reused by heat sterilization treatment or the like.

When the introduction fluid machine 51 is a sealed pump, as shown in Fig.1, the introduction fluid machine 51 has a pump head 151 and a drive unit 251 such as a motor. The pump head 151 and the drive unit 251 are removable. The pump head 151 includes rollers which squeeze the culture medium channel, such as a tube, from the exterior. The drive unit 251 rotates the rollers of the pump head 151. When the discharge fluid machine 52 is a sealed pump, the discharge fluid machine 52 has a pump head 152 and a drive unit 252 such as a motor. The pump head 152 and the drive unit 252 are removable. The pump head 152 includes rollers which squeeze the culture medium channel, such as a tube, from the exterior. The drive unit 252 rotates the rollers of the pump head 152.

As shown in Fig.3, culture medium channel 200 may be provided with a culture medium tank 60 into which the culture medium can enter. The culture medium entering the culture medium tank 60 from the culture medium channel 200 flows out back into the culture medium channel 200. By providing the culture medium tank 60, it is possible to increase the amount of the culture medium circulating through the culture medium channel 200 and the culture medium holding chamber 40.

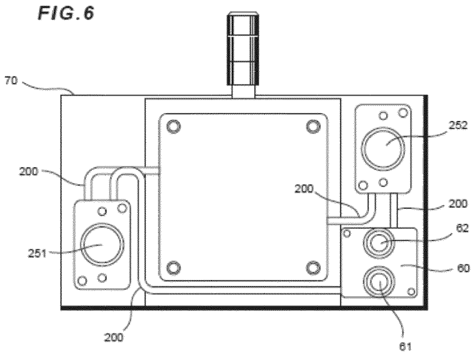

The culture medium tank 60 may be provided with: a feed port for supplying fluid into the culture medium tank 60; and a discharge port for discharging the fluid in the culture medium tank 60. For example, a plug 61 shown in Fig.6 is inserted into the feed port of the culture medium tank 60 to which a feeder such as bags, bellows and syringes for supplying fluids can be connected. The feeder may be a fluid machine such as a pump. However, the feeder may be directly connected to the feed port of the culture medium tank 60. The feeder is detachable from the feed port, and when the feeder is not connected to the feed port, the feed port is sealable, and no fluid exchange occurs between the culture medium channel 200 and the outside through feed port. Alternatively, the feed port is shielded from the outside by being connected to the feeder. The plug 61 may be a needleless connector. The needleless connector may be split-septum type or mechanical valve type. Even when the feeder or the flow path connected to feeder is inserted into the plug 61, the outside air does not enter the culture medium tank 60 through the plug 61. However, the plug 61 may be a connector into which a needle is inserted.

Further, inserted into the discharge port of the culture medium tank 60 is a plug 62 to which a discharger such as bags, bellows and syringes for draining fluids in the culture medium tank 60 can be connected. The discharger may be fluid machines such as pumps. However, the discharger may be directly connected to the discharge port of the culture medium tank 60. The discharger may actively aspirate fluid in the culture medium channel. Alternatively, the discharger may passively increase the internal volume in response to the pressure in culture medium channel and receive the fluid extruded from culture medium channel. When the discharger is detachable from the discharge port, and not connected to the discharge port, the discharge port is sealable, and there is no changeover of fluids in or out of the culture medium channel 200 via the discharge port. Alternatively, the discharge port is shielded from the outside by being connected to the discharger. The plug 62 may be a needleless connector. The needleless connector may be split-septum type or mechanical valve type. Even when the discharger or the flow path connected to the discharger is inserted into the plug 62, the outside air does not enter the culture medium tank 60 through the plug 62. However, the plug 62 may be a connector into which a needle is inserted.

For example, when the culture medium holding chamber 40 shown in Fig.1 is in close contact with the culture chamber 30 with the culture medium side plate 22, the culture component permeable member 10, and the culture side plate 21 interposed therebetween, and air is contained in the culture medium holding chamber 40, the culture medium channel 200, and culture medium tank 60 shown in Fig.3, the culture medium can be introduced into the culture medium holding chamber 40, the culture medium channel 200, and the culture medium tank 60 by injecting the culture medium from the feed port of the culture medium tank 60 into the culture medium holding chamber 40, the culture medium channel 200, and the culture medium tank 60, while exhausting air in the culture medium holding chamber 40, the culture medium channel 200, and the culture medium tank 60 through the discharge port of the culture medium tank 60. The air layer in the culture medium holding chamber 40, the culture medium channel 200 and the culture medium tank 60 may be completely eliminated, or an air layer may be remained.

A feeder filled with culture medium and an empty discharger may be connected to the culture medium channel 200, and a fluid machine may be driven to introduce culture medium from the feeder into the culture medium channel 200 to introduce air into the discharger. At this time, the culture medium may be actively injected into the culture medium channel 200 by the feeder, or the culture medium in the feeder may be sucked into the culture medium channel 200 which has become low in pressure by the driving of the fluid machine, and the inner volume of the feeder may be passively reduced. Further, the discharger may actively suck air in the culture medium channel 200, or the air in the culture medium channel 200, which has become high in pressure due to the driving of the fluid machine, may flow into the discharger, and the inner volume of the discharger may passively be increased.

Further, when the culture medium is present in the culture medium holding chamber 40, the culture medium channel 200 and the culture medium tank 60, the cell culture medium can be replaced in the culture medium tank 60 by injecting the culture medium into the culture medium tank 60 from the feed port of the culture medium tank 60 while the culture medium in the culture medium tank 60 is discharged from the discharge port of the culture medium tank 60.

A feeder filled with culture medium and an empty discharger may be connected to culture medium channel 200, and a fluid machine may be driven to introduce a new culture medium from the feeder into the culture medium channel 200, and the old culture medium may be introduced into the discharger. At this time, a new culture medium may be actively injected into the culture medium channel 200 by the feeder, or a new culture medium in the feeder may be sucked into the culture medium channel 200 which has become low in pressure by the driving of the fluid machine, and the inner volume of the feeder may be passively reduced. Further, the discharger may actively suck the old culture medium in the culture medium channel 200, or the old culture medium in the culture medium channel 200, which has become high in pressure due to the driving of the fluid machine, may flow into the discharger, and the inner volume of the discharger may passively be increased.

The feed port for supplying the culture medium into the culture medium channel 200 and the culture medium holding chamber 40, and the discharge port for discharging air in the culture medium channel 200 and the culture medium holding chamber 40 may be provided at a portion of the culture medium channel 200 other than where the culture medium tank 60 is provided. For example, the feed port for supplying the culture medium into the culture medium channel 200 and the culture medium holding chamber 40, and the discharge port for discharging air in the culture medium channel 200 and the culture medium holding chamber 40 may be provided at the culture medium channel 200.

The cell culture vessel according to the embodiment may include a temperature controller for heating and cooling at least one of the culture medium holding chamber 40, the culture medium channel 200, and the culture medium tank 60. By adjusting the temperature of any one of the culture medium holding chamber 40, the culture medium channel 200, and the culture medium tank 60 by the temperature controller, it is possible to adjust the temperature of the culture medium. The cell culture vessel according to the embodiment may further include a thermometer for measuring the temperature of the culture medium. The thermometer may measure the temperature of the culture medium based on at least one of the temperature of the culture medium holding chamber 40, the culture medium channel 200, and the culture medium tank 60 without contacting the culture medium, or may directly measure the temperature of the culture medium. In this case, the temperature controller may be feedback-controlled so that the temperature of the culture medium becomes a predetermined temperature.

As shown in Fig.3, the culture medium holding chamber 40, the culture medium channel 200, the pump head 151, the pump head 152, and the culture medium tank 60 may be stored in the channel case 70. In the channel case 70, the culture medium holding chamber 40, the culture medium channel 200, the pump head 151, the pump head 152, and the culture medium tank 60 may be completely embedded in a gas impermeable material. The culture medium channel 200 may be tunneled in the gas impermeable material. For example, the channel case 70 is provided with a hole for inserting a shaft into the pump head 151, a hole for inserting a shaft into the pump head 152, a hole for inserting the plug 61 into the feed port of the culture medium tank 60, and a hole for inserting the plug 62 into the discharge port of the culture medium tank 60. The hole for inserting the plug 61 into the feed port of the culture medium tank 60 and the hole for inserting the plug 62 into the discharge port of the culture medium tank 60 may be closable.

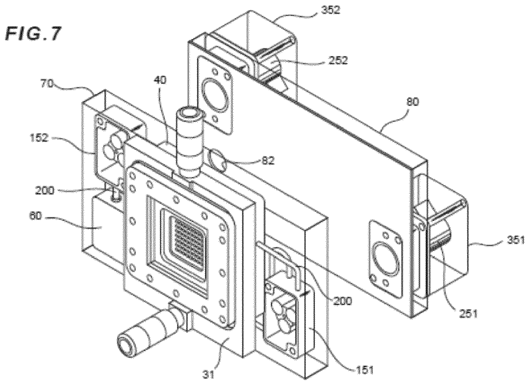

As shown in Fig.7, the drive unit 251 of the introduction fluid machine 51 and the drive unit 252 of the discharge fluid machine 52 may be disposed on a substrate-shaped drive unit holding member 80. The drive unit holding member 80 is provided with a hole for inserting the plug 61 into the feed port of the culture medium tank 60 and a hole 82 for inserting the plug 62 into the discharge port of the culture medium tank 60. The hole for inserting the plug 61 into the feed port of the culture medium tank 60 and the hole 82 for inserting the plug 62 into the discharge port of the culture medium tank 60 may be closable.

The drive unit holding member 80 is brought into close contact with the channel case 70 via a packing 90 shown in Fig.1. The packing 90 suppresses the air from entering the channel case 70 from the contact portion of the channel case 70 and the drive unit holding member 80.

The fluid machine for introducing the culture medium into the culture medium holding chamber 40 and for draining the culture medium from within the culture medium holding chamber 40 may be covered with an outside air blocking member for fluid machine. The outside air blocking member for fluid machine may have an outside air blocking member for introduction fluid machine 351 covering the drive unit 251 of the introduction fluid machine 51 disposed on the drive unit holding member 80, and an outside air blocking member for discharge fluid machine 352 covering the drive unit 252 of the discharge fluid machine 52, for example, as shown in Fig.7.

The channel case 70 and the drive unit holding member 80 are detachable. When the drive unit holding member 80 is brought into close contact with the channel case 70; the hole for inserting the plug 61 into the feed port of the culture medium tank 60, and the hole for inserting the plug 62 into the discharge port of the culture medium tank 60 are closed; the drive unit 251 of the introduction fluid machine 51 is covered with the outside air blocking member for introduction fluid machine 351; and the drive unit 252 of the discharge fluid machine 52 is covered with the outside air blocking member for discharge fluid machine 352; the inside of the channel case 70 is blocked from the outside air, and the outside air can not enter the channel case 70. Therefore, exchange of gas inside and outside the channel case 70 does not occur. Therefore, the outside air does not enter into the culture medium holding chamber 40 and the culture medium channel 200. By blocking the inside of the channel case 70 constituting at least a part of the outside air blocking member for culture medium channel from the outside air, it is possible to suppress the change of the pH of the culture medium in the culture medium holding chamber 40 and the culture medium channel 200, and to keep it within a predetermined range even when the culture medium channel 200 is a gas permeable tube.

It is to be noted that, according to the findings of the present inventors, since the cells can be cultured in a completely closed sealed space, it is not necessary to actively supply carbon dioxide gas, nitrogen gas, oxygen gas, and the like into the culture medium holding chamber 40 and the culture medium channel 200. Therefore, the culture medium holding chamber 40 and the culture medium channel 200 do not have to be disposed in a CO₂ incubator. Further, since cells, microorganisms, viruses, dust, and the like existing outside the culture medium holding chamber 40 and the culture medium channel 200 do not enter into the sealed culture medium holding chamber 40 and culture medium channel 200, the cleanliness in the culture medium holding chamber 40 and the culture medium channel 200 is maintained. Therefore, it is not necessary to place the culture medium holding chamber 40 and the culture medium channel 200 in a clean room. The culture medium holding chamber 40 may be embedded within a gas impermeable material. In other words, the culture medium holding chamber 40 may be embedded in the gas impermeable material.

When the drive unit holding member 80 is removed from the channel case 70, by closing the hole of the channel case 70 for inserting the plug 61 into the feed port of the culture medium tank 60 and the hole of the channel case 70 for inserting the plug 62 into the discharge port of the culture medium tank 60, the inside of the channel case 70 is sealed, and it is possible to suppress the material inside the channel case 70 to flow out or the outside air to enter the channel case 70.

The channel case 70 including the culture medium channel 200 and the pump heads 151, 152 inside is disposable. On the other hand, the drive unit holding member 80 holding the drive units 251 and 252 can be repeatedly used.

For example, the introduction fluid machine 51 and the discharge fluid machine 52 are controlled so that the amount of culture medium delivered into the culture medium holding chamber 40 by the introduction fluid machine 51 shown in Fig.2 is the same as the amount of culture medium discharged from the culture medium holding chamber 40 by the discharge fluid machine 52. The introduction fluid machine 51 and the discharge fluid machine 52 may constantly feed culture medium into the culture medium holding chamber 40, or may feed culture medium at an appropriate interval.

When the culture medium is constantly fed into the culture medium holding chamber 40, the flow rate of the culture medium delivered into the culture medium holding chamber 40 may be constant or not constant. For example, the culture medium and the cell masses in the culture medium may be monitored by a photographing device, and the flow rate of the culture medium delivered into the culture medium holding chamber 40 may be increased or decreased depending on the condition of the culture medium and the cell masses in the culture medium.

Further, the culture medium may be not continuously fed into the culture medium holding chamber 40, and the feeding of the culture medium may be started and terminated depending on, for example, the state of the culture medium, the state of the cell masses in the culture medium, the number of cells, the number of cell masses, the turbidity of culture medium, and the change of the pH. Again, depending on the condition of the culture medium and the cell masses in the culture medium, the flow rate of the culture medium to be delivered may be increased or decreased.

In the culture medium during agitation, cells may randomly collide with each other and bind to form cell masses (colonies) of various sizes. Consequently, homogeneity between colonies may not be maintained. Moreover, in colonies that are too large, nutrients and growth factors may not reach the inside of the colony, resulting in differentiation and cell death from the inside of the colony. On the other hand, colonies that are too small may not be suitable for subculture. In contrast, in the culture chamber 30 shown in Fig.2, the flow rate of the culture medium is slow, since the culture medium does not flow, and the frequency of the cells colliding with each other is low. Therefore, it is possible to maintain the clonality in the colonies. Thus, for example, when the cell is a stem cell, such as an iPS cell, it is possible to ensure the clonality of the stem cells deriving from one cell. Further, since the frequency of collision between stem cells is low, it is possible to keep the size of the colonies of the stem cells uniform.

The cell culture vessel according to the embodiment may further include a photographing device such as a photographic camera or a video camera for taking the culture medium containing cells in the culture chamber 30 via the window 132 of the cover 32 of the culture chamber 30.

According to the cell culture vessel of the embodiment, for example, since the cells are cultured in a complete sealed system, the risks of cross contamination due to leakage of the cells from the culture device can be reduced. Further, for example, even if the cells are in infection with viruses, such as HIV-hepatitis viruses, the risks of infection to the operator due to cell leakage can be reduced. Further, the risks of the culture medium in the cell culture vessel being contaminated with airborne bacteria, viruses, molds, etc. outside cell culture vessel can be reduced. Furthermore, according to the cell culture vessel of the embodiment, cells can be cultured without using a CO₂ incubator.

It is to be noted that, for example, when circulation of the culture medium is not necessary, it is not necessary to connect the culture medium channel 200 to the culture medium holding chamber 40 shown in Fig.3. Further, the cells may be subjected to floating culture or adherent culture in the culture chamber 30. When the cells are subjected to adherent culture, the surface of the culture side plate 21 shown in Fig.1 may be cell adherent, or the surface of the culture component permeable member 10 may be cell adherent. Further, in the culture chamber 30 of the cell culture vessel according to the embodiment, cells may be induced while culturing. Further, the culture medium channel may be used without being connected to the culture medium holding chamber or the culture chamber, and the cells may be cultured or induced in the sealed system channel.

The sealed system is not limited to the cell culture vessel shown in Figs. 1-7. For example, the sealed system may be a container. The container may be a tube or flask. The container may be made of resin or made of glass. In order to completely close the inside of the container, the periphery of the cap, the lid, or the like of the container may be wound with a film such as a paraffin film.

### [EXAMPLES]

### (Example 1)

A stem cell culture medium (DMEM/F12 with 20%KnockOut SR(R) (ThermoFisher SCIENTIFIC) was gelled to create a gel culture medium. The pH of the gel culture medium was adjusted to between 4.0 and 10.0. To the gel culture medium was added 2×10⁵ cells/mL of iPS cells as single cells or cell masses. The gel culture medium containing the iPS cells was placed in a 15 mL Falcon tube(R) (Corning). Then, the caps of some of the Falcon tubes were firmly tightened and the periphery of the Falcon tubes and the caps was wrapped with a paraffin film (Parafilm(R), Bemis) to isolate the inside of the Falcon tubes from the outside air and completely prevent gas (air) in the Falcon tubes from being exchanged with the outside air. As to the other Falcon tubes, the caps were only tightened and not wrapped with paraffin film.

The Falcon tubes which were not wrapped with paraffin film were placed in an incubator at 37 °C and carbon dioxide concentration of 5% to initiate floating culture of iPS cells. Further, the Falcon tubes which were wrapped with paraffin film were placed in a constant temperature bath at 37 °C without being placed in a CO₂ incubator, and floating culture of iPS cells was initiated. As a constant temperature bath, a bead bath, a water bath, and a thermostat capable of electronically controlling the temperature was used. The constant temperature bath was placed in the laboratory and was not shielded from the air in the laboratory. Thereafter, once every 2 days, the caps of the respective Falcon tubes were opened, and 2 mL of gel culture medium with a pH of between 4.0 and 10.0 was added into the falcon tubes. After the addition of the gel culture medium, the Falcon tubes in which the caps were tightened and placed in the constant temperature bath were wrapped around at the periphery of the caps with paraffin film as described above.

7 to 10 days after initiating culture in the Falcon tubes, the caps of the Falcon tubes were opened, and the cell masses of iPS cells formed in the gel culture medium were harvested using filters, washed with PBS, and placed in Falcon tubes. Further, 500 µL of cell dissociation reagent (TrypLE Select(R), Thermo Fisher) was added to the cell masses, and the cell masses were incubated for 5 minutes in a CO₂ incubator. Next, the Falcon tubes were taken out from the incubator, and 500 µL of stem cell culture medium (DMEM/F12 containing 20% KnockOut SR(R), ThermoFisher SCIENTIFIC) was placed in the Falcon tube, and the cell masses was suspended to dissociate the iPS cells into single cells. 2 mL of stem cell culture medium (DMEM/F12 with 20% KnockOut SR(R), ThermoFisher SCIENTIFIC) was added into Falcon tubes, and the Falcon tubes were centrifuged at 200g using a centrifuge. After centrifugation, the supernatant in the Falcon tubes was removed, and the iPS cells were placed in Falcon tubes with gel culture medium. Thereafter, as described above, iPS cells were subjected to floating culture in the sealed Falcon tubes for 7 to 10 days while adding gel culture medium once every 2 days.

Thereafter, as described above, passage and floating culture from 7 to 10 days were repeated, and the iPS cells were subjected to floating culture in the sealed Falcon tubes for a total of 1 month or more.

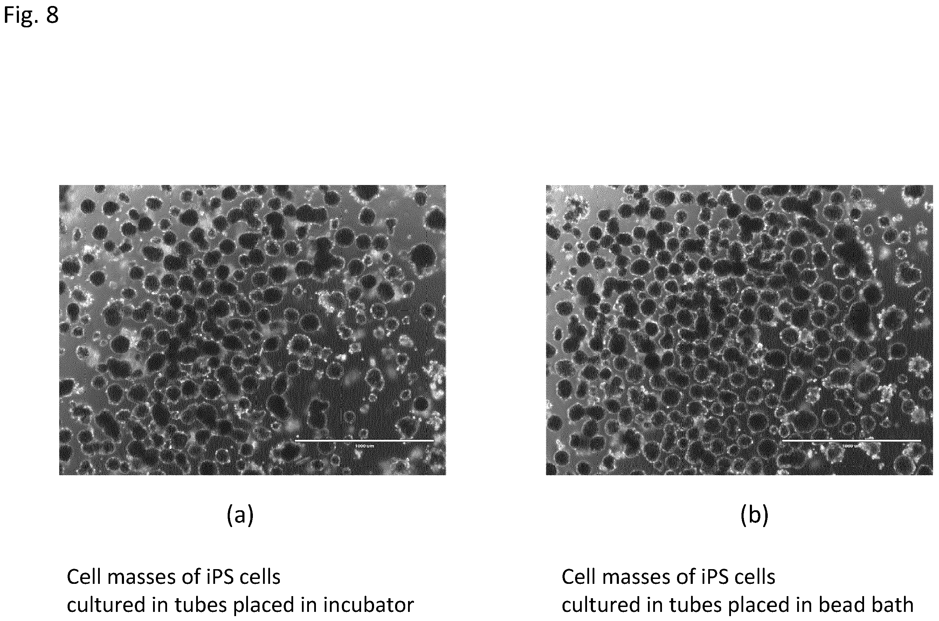

The iPS cells cultured by placing the Falcon tubes in an incubator and the iPS cells cultured by placing the Falcon tubes in a bead bath were observed under a microscope, and were confirmed that all of them formed a uniform cell mass as shown in Fig.8. Similar results were obtained for iPS cells cultured in the Falcon tubes placed in a constant temperature bath other than a bead bath.

Further, at passage, some of single cell iPS cells were dispensed, and the iPS cells were fixed using 4% paraformaldehyde. Furthermore, the expression level of cell surface antigen TRA-1-60 in the immobilized iPS cells was measured using a flow cytometer. TRA-1-60 is a typical surface antigen of pluripotent stem cells and is known to have reduced expression levels in differentiated cells.

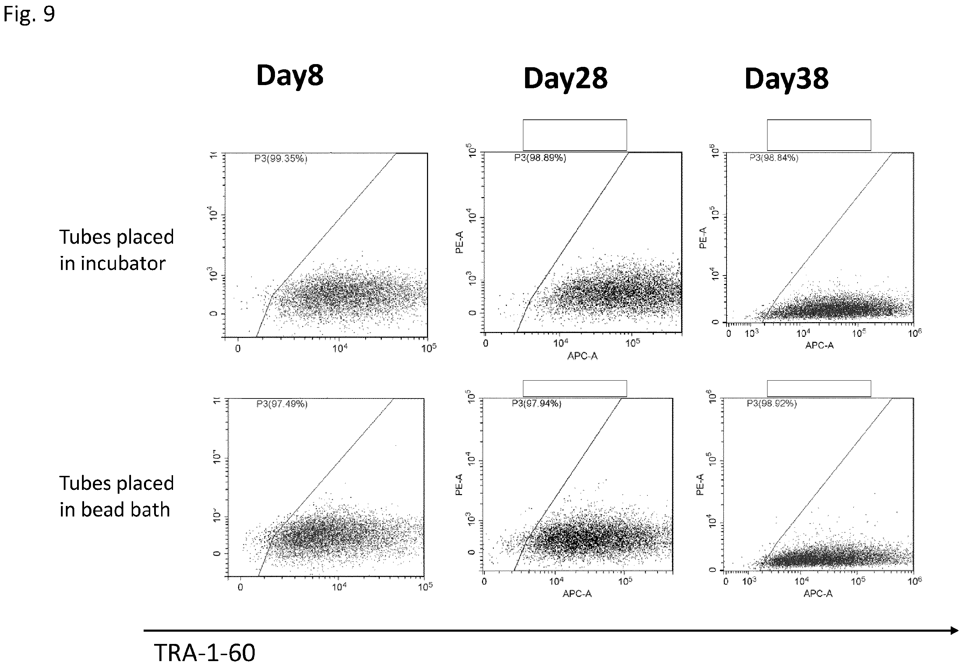

As a result, as shown in Fig.9, the iPS cells cultured by placing the Falcon tubes in an incubator and the iPS cells cultured by placing the Falcon tubes in a bead bath were almost 100% TRA1-60 positive at 8 days, 28 days, and 38 days from the initiation of culture. Similar results were obtained for iPS cells cultured in the Falcon tubes placed in a constant temperature bath other than a bead bath. Thus, it has been shown that, when the vessel is sealed to form a sealed system, the stem cells can be cultured for a long period of time while maintaining their pluripotency in an undifferentiated state, without controlling the carbon dioxide concentration in the vessel.

### (Example 2)

A gel culture medium was prepared in the same manner as in Example 1. 2×10⁵ cells/mL of iPS cells dissociated into single cells were added to the gel culture medium. 2 mL of gel culture medium containing iPS cells was placed in a 2 mL gas impermeable tube with rubber packing so that no air layers was remained inside. Thereafter, the cap of the tube was firmly tightened, and the periphery of the tube and the cap was wrapped around with paraffin film to shield the inside of the tube from the outside air and prevent the outside air from entering the tube. This prevented the gel culture medium from contacting the gas (air) layer during culture.

The respective tubes to which the paraffin film has been wrapped was placed in a 37 °C CO₂ incubator and a 37 °C constant temperature bath outside the CO₂ incubator to initiate floating culture of the iPS cells. As a constant temperature bath, a bead bath, a water bath, and a thermostat capable of electronically controlling the temperature was used. The constant temperature bath was placed in the laboratory and was not shielded from the air in the laboratory. No culture medium was added or replaced during culture. 10 to 11 days after initiating the culture in the tube, the cap of the tube was opened, and the cell masses of iPS cells formed in the gel culture medium was harvested using filters, washed with PBS, and placed in a tube. Further, 500 µL of cell dissociation reagent (TrypLE Select(R), Thermo Fisher) was added to the cell masses, and the cell masses were incubated for 5 minutes in a CO₂ incubator. Next, the tube was taken out from the incubator, and 500 µL of stem cell culture medium (DMEM/F12 containing 20% KnockOut SR(R), ThermoFisher SCIENTIFIC) was placed in the tube, and the cell masses was suspended to dissociate the iPS cells into single cells. 2 mL of stem cell culture medium (DMEM/F12 containing 20% KnockOut SR(R), ThermoFisher SCIENTIFIC) was added into the tube, and the tube was centrifuged at 200g using a centrifuge. After centrifugation, the supernatant in the tube was removed, and gel culture medium was placed in the tube so that the number of iPS cells was 2×10⁵ cells/mL. Then, as described above, iPS cells were subjected to floating culture in the sealed tube for 5 to 11 days without addition or replacement of gel culture medium.

Thereafter, as described above, passage and floating culture from 5 to 11 days were repeated, and the iPS cells were subjected to floating culture in the sealed tube for a total of 1 month or more.

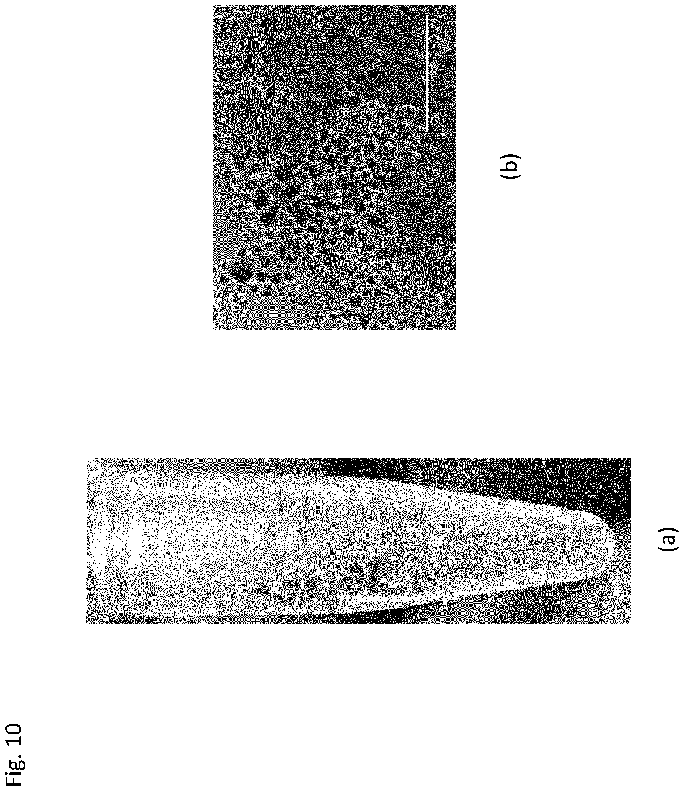

The iPS cells cultured by placing the tube in an incubator were observed by a camera and a microscope, and were confirmed that a uniform cell mass was formed as shown in Fig.10. Similar results were obtained with iPS cells cultured in tubes placed in a constant temperature bath outside the incubator.

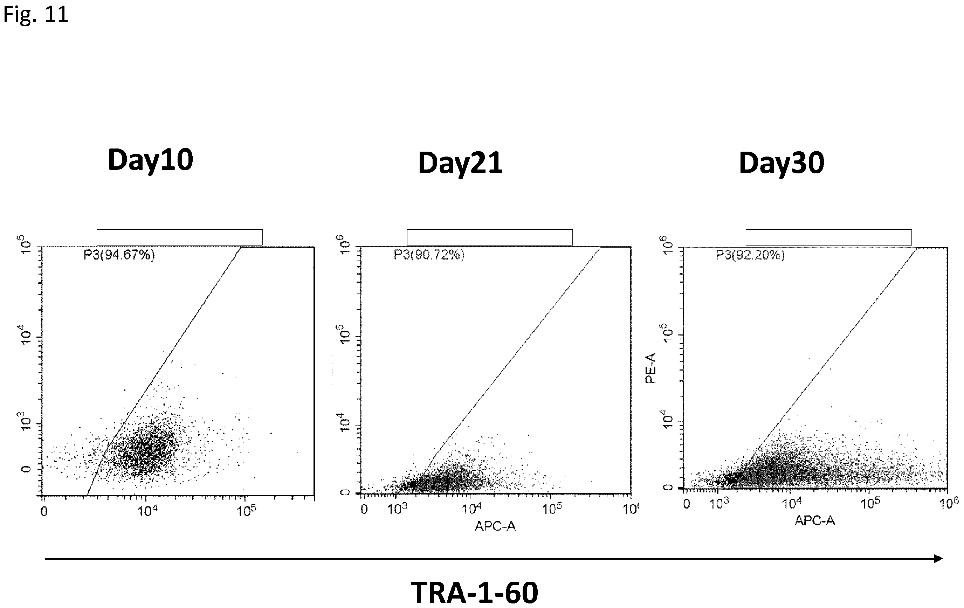

Further, at passage, some of single cell iPS cells were dispensed, and the iPS cells were fixed using 4% paraformaldehyde. Furthermore, the expression level of cell surface antigen TRA-1-60 in the immobilized iPS cells was measured using a flow cytometer. As a result, as shown in Fig.11, the iPS cells cultured by placing the tubes in an incubator were more than 90% positive for TRA1-60 at 10 days, 21 days, and 30 days from the initiation of culture. Similar results were obtained with iPS cells cultured in tubes placed in a constant temperature bath outside the incubator. Thus, it has been shown that, when the vessel is sealed, the stem cells can be cultured for a long period of time while maintaining their pluripotency in undifferentiated state, without controlling the carbon dioxide concentration and without adding or replacing the culture medium in the vessel.

### (Example 3)

A gel culture medium was prepared in the same manner as in Example 1. iPS cells dissociated into single cells were added to the gel culture medium. 2 mL of gel culture medium containing the iPS cells was placed in a 15 mL Falcon tube. Then, the cap of the Falcon tube was firmly tightened.

The Falcon tube was placed in a CO₂ incubator at 37 °C to initiate floating culture of the iPS cells. Thereafter, once every 2 days, the caps of the Falcon tubes were opened, and 2 mL of gel culture medium with a pH of between 4.0 and 10.0 was added into the falcon tubes. After the addition of the gel culture medium, the cap was tightened as described above.

7 to 10 days after initiating culture in the Falcon tubes, the caps of the Falcon tubes were opened, and the cell masses of iPS cells formed in the gel culture medium were harvested using filters, washed with PBS, and placed in Falcon tubes. Further, 500 µL of cell dissociation reagent (TrypLE Select(R), Thermo Fisher) was added to the cell masses, and the cell masses were incubated for 5 minutes in a CO₂ incubator. Next, the Falcon tube was taken out from the incubator, 500 µL of culture medium containing 20%KnockOut SR(R) (ThermoFisher SCIENTIFIC), GlutaMAX(R) (ThermoFisher SCIENTIFIC), and non-essential amino acids (NEAA) was placed in the Falcon tube, and the cell masses were suspended to dissociate the iPS cells into single cells. 2 mL of stem cell culture medium (DMEM/F12 with 20% KnockOut SR(R), ThermoFisher SCIENTIFIC) was added into Falcon tubes, and the Falcon tubes were centrifuged at 200g using a centrifuge. After centrifugation, the supernatant in the Falcon tube was removed, and the gel culture medium was placed in a Falcon tube so that the number of iPS cells was 2×10⁵ cells/mL. Thereafter, as described above, iPS cells were subjected to floating culture in the sealed Falcon tubes for 7 to 10 days while adding gel culture medium once every 2 days.

Thereafter, as described above, passage and floating culture from 7 to 10 days were repeated, and the iPS cells were subjected to floating culture in the sealed Falcon tubes for a total of 1 month or more.

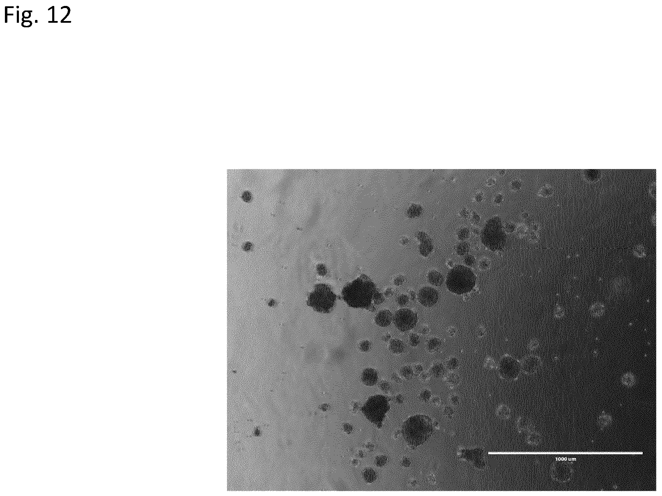

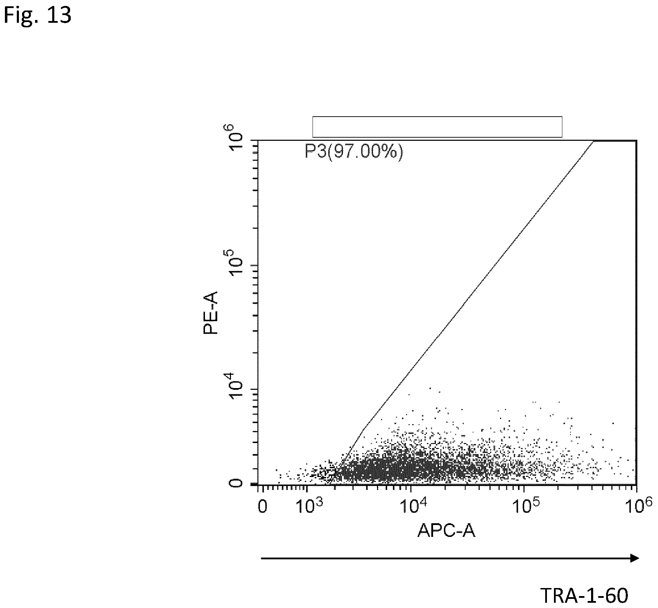

The iPS cells cultured in the Falcon tube were observed under a microscope, and were confirmed that each formed cell masses as shown in Fig.12. Further, the expression level of cell surface antigen TRA-1-60 in the iPS cells was measured using a flow cytometer in the same manner as in Example 3, the iPS cells were almost 100% TRA-1-60 positive at 7 days to 21 days from the initiation of culture, as shown in Fig.13.

### (Example 4)

Growth factors were added to the culture medium (StemSpan H3000(R), STEMCELL Technologies Inc.) and deacylated gellan gum was further added to the culture medium to prepare the gel culture medium.

The prepared gel culture medium was placed in a 15 mL tube and the gel culture medium was seeded with 2×10⁵ of hemocytes (monocytes). Then, the 15 ml tube was placed in a 37 °C CO₂ incubator and the hemocytes were cultured for 7 days. Thereafter, Sendai virus vectors (CytoTune-iPS2. 0, ID Pharma Co., Ltd.) carrying OCT3/4, SOX2, KLF4, cMYC was added to the gel culture medium to subject the hemocytes to infection with Sendai viruses so that the multiplicity of infection (MOI) is 10.0.

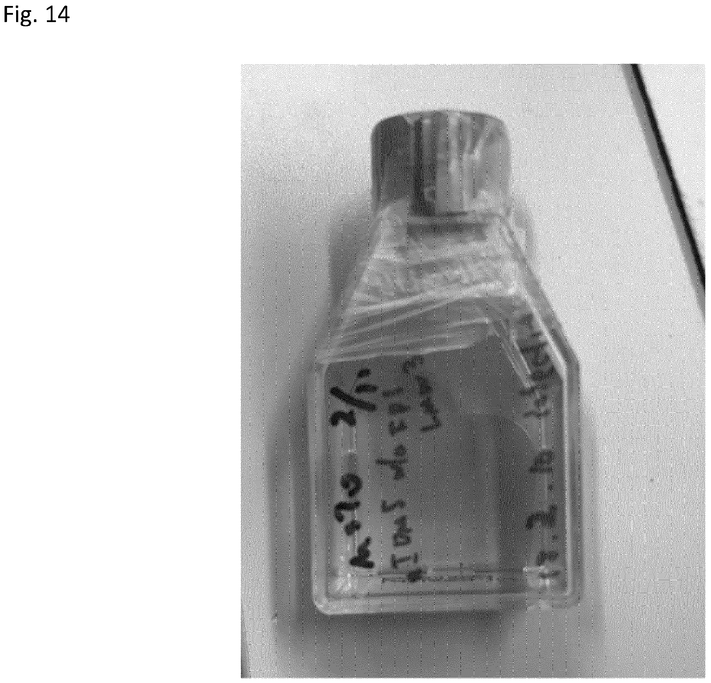

After the Sendai viruses were added to the gel culture medium, 30 mL of stem cell culture medium (DMEM/F12 containing 20%KnockOut SR(R), ThermoFisher SCIENTIFIC) was added to the gel culture medium, the culture medium containing cells infected with Sendai viruses was placed in a flask seeded with feeder cells, and the flask was allowed to stand for 15 days to subject the cells infected with Sendai viruses to adherent culture. There was no air layer in the flask. During that time, as shown in Fig.14, the periphery of the cap of the flask was wound with paraffin film to completely close the inside of the flask, and no culture medium exchange and no gas exchange were performed while no control of CO₂ concentration in the flask was performed.

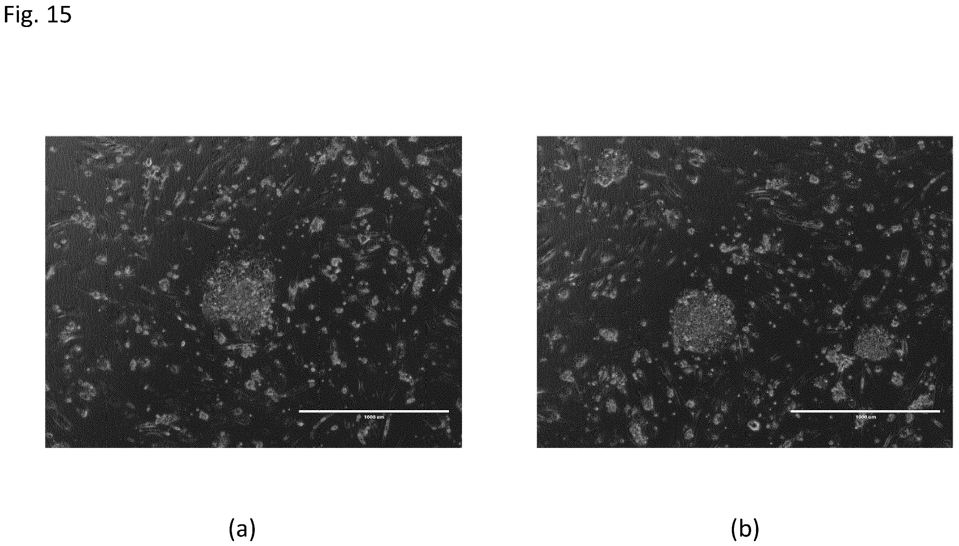

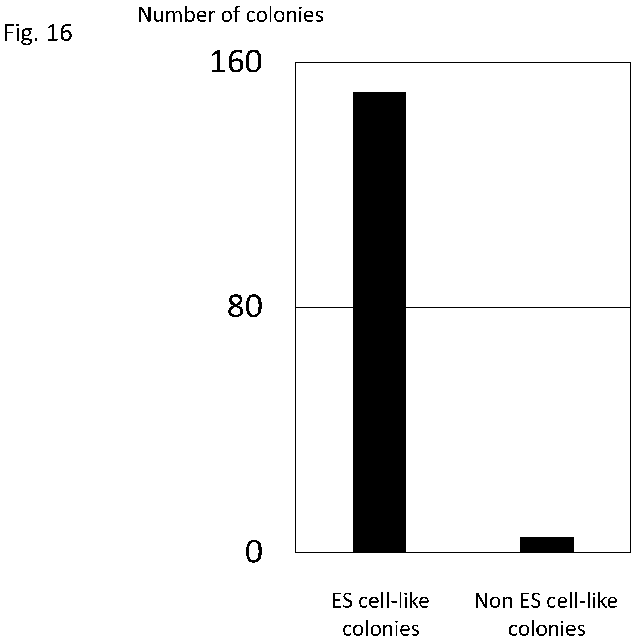

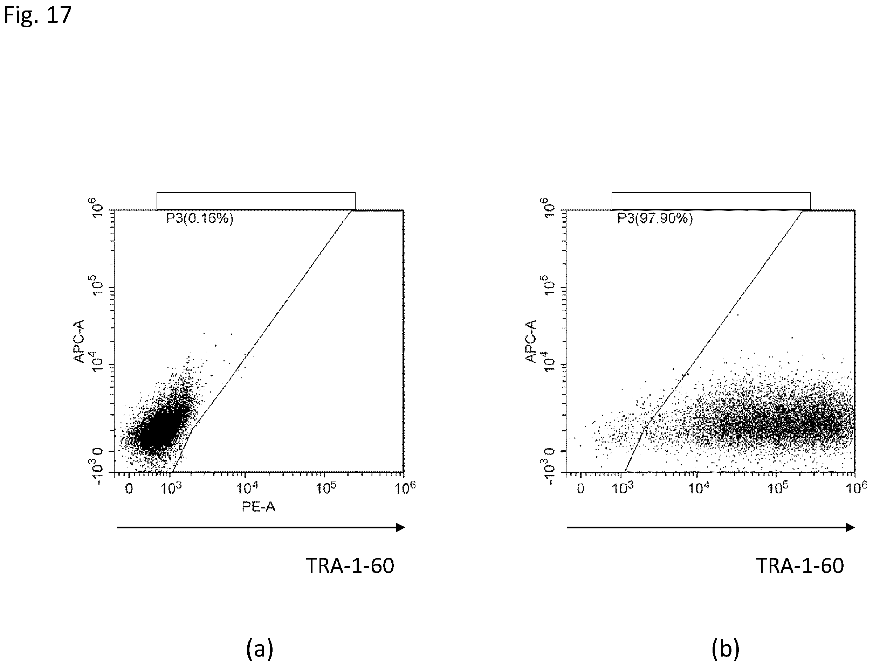

After 15 days, the cells were observed under a microscope, and it was confirmed that they formed ES cell-like colonies, as shown in Fig.15. As shown in Fig.16, nearly 100% of the colonies were ES cell-like colonies. Further, the cells were immobilized using 4%-paraformaldehyde and the expression level of cell surface antigen TRA-1-60 in the immobilized cells was measured using a flow cytometer, and it was confirmed that, as shown in Fig.17(a), the cells before induction were nearly 100% TRA-1-60 negative, while as shown in Fig.17(b), the cells after induction were nearly 100% TRA-1-60 positive, thus almost completely reprogrammed. Thus, it has been shown that iPS cells can be derived from cells other than stem cells in a completely closed environment without culture medium exchanges and gas exchanges.

### (Example 5)

A gel culture medium prepared in the same manner as in Example 4 was placed in a 15 mL tube, and 2×10⁵ of hemocytes (monocytes) were seeded in the gel culture medium. Then, the 15 ml tube was placed in a 37 °C CO₂ incubator and the hemocytes were cultured for 7 days. Thereafter, Sendai virus vectors (CytoTune-iPS2. 0, ID Pharma Co., Ltd.) carrying OCT3/4, SOX2, KLF4, cMYC was added to the gel culture medium to subject the hemocytes to infection with Sendai viruses so that the multiplicity of infection (MOI) is 10.0.

After adding Sendai viruses to the gel culture medium, 15 mL of gelled stem cell culture medium (DMEM/F12 containing 20%KnockOut SR(R), ThermoFisher SCIENTIFIC) was added to the gel culture medium, 15 mL of the culture medium containing cells infected with Sendai viruses was placed in a 15 mL tube, and the 15 mL tube was allowed to stand for 15 days to subject the cells infected with Sendai viruses to floating culture. There was no air layer in the 15 mL tube. During that time, the inside of the 15 mL tube was completely closed, and no culture medium exchange or gas exchange was performed, while no control of CO₂ concentration in the 15 mL tube was performed.

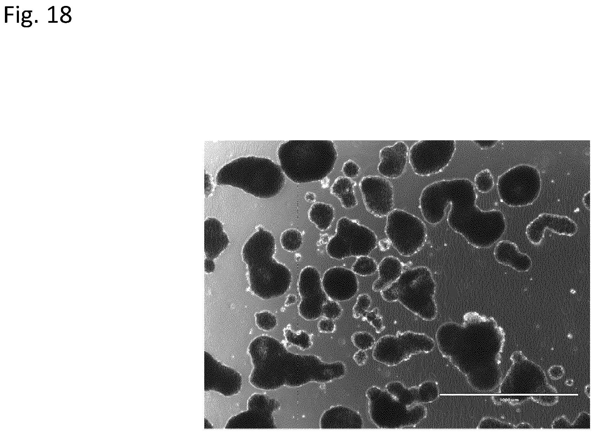

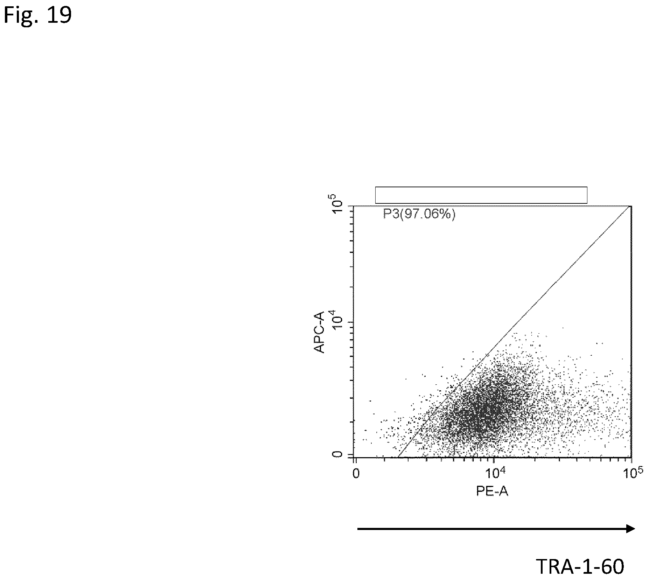

After 15 days, the cells were observed under a microscope, and it was confirmed that they formed ES cell-like colonies, as shown in Fig.18. Further, the cells were immobilized using 4%-paraformaldehyde and the expression level of cell surface antigen TRA-1-60 in the immobilized cells was measured using a flow cytometer, and it was confirmed that, as shown in Fig.19, the expression level was almost 100% TRA-1-60 positive, thus almost completely reprogrammed. Thus, it has been shown that iPS cells can be derived from cells other than stem cells in a completely closed environment without culture medium exchanges and gas exchanges.

### (Example 6)

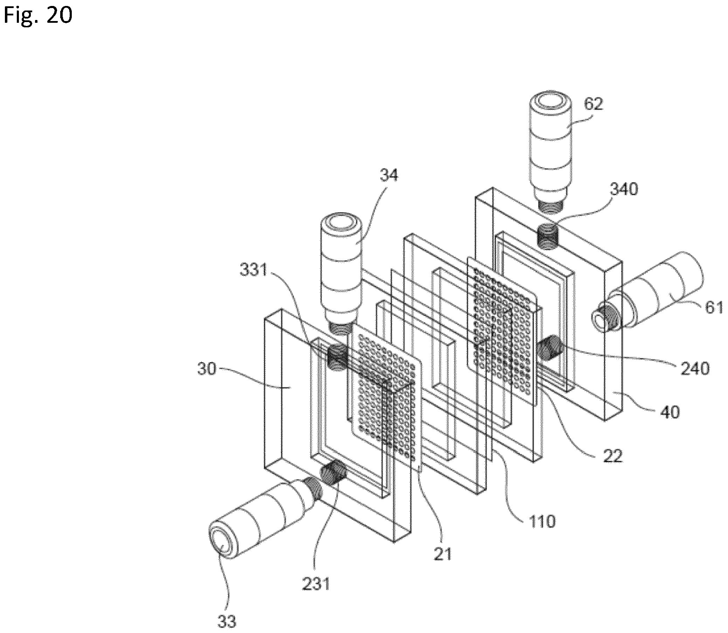

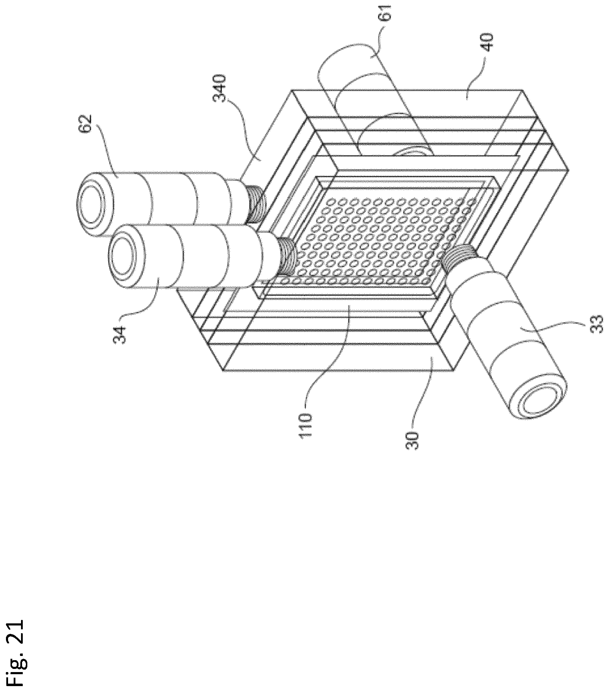

As shown in Fig.20 and Fig.21, a semipermeable membrane 110 (Asahi Kasei Co., Ltd. or SPECTRUM) was sandwiched between the culture side plate 21 and the culture medium side plate 22, and further, the semipermeable membrane 110, the culture side plate 21 and the culture medium side plate 22 were sandwiched between the culture chamber 30 and the culture medium holding chamber 40.

A stem cell culture medium (reprocell) containing 20% of alternative serum (KnockOut SR(R), Gibco) was gelled to prepare a gel culture medium. 2×10⁵ cells/mL of iPS cells dissociated into single cells were added to the gel culture medium to prepare a culture medium containing cells.

The culture medium containing cells was placed in a syringe, and the syringe was connected to the feed port 231 of the culture chamber 30 via the plug 33. Further, an empty syringe was connected to the discharge port 331 of the culture chamber 30 via the plug 34. Next, the culture medium containing cells in the syringe was injected into the culture chamber 30 from the feed port 231 of the culture chamber 30. Due to the pressure increase in the culture chamber 30, the piston of the syringe connected to the discharge port 331 was passively rised, and the air in the culture chamber 30 moved into the syringe connected to the discharge port 331 of the culture chamber 30. The culture medium containing cells was injected into the culture chamber 30 until the air layer in the culture chamber 30 was completely eliminated. Thereafter, the feed port 231 and the discharge port 331 of the culture chamber 30 were shielded.

The gel culture medium was placed in a syringe, and the syringe was connected to the inlet 240 of the culture medium holding chamber 40 via the plug 61. Further, an empty syringe was connected to the discharge port 340 of the culture medium holding chamber 40 via the plug 62. Next, the gel culture medium in the syringe was injected into the culture medium holding chamber 40 from the inlet 240 of the culture medium holding chamber 40. Due to the pressure increase in the culture medium holding chamber 40, the syringe connected to the discharge port 340 of the culture medium holding chamber 40 was passively rised, and the air in the culture medium holding chamber 40 moved into the syringe connected to the discharge port 340 of the culture medium holding chamber 40. The gel culture medium was injected into the culture medium holding chamber 40 until the air layer in the culture medium holding chamber 40 was completely eliminated. Thereafter, the inlet 240 and the discharge port 340 of the culture medium holding chamber 40 were shielded. Thus, the inside of the culture chamber 30 and the culture medium holding chamber 40 was sealed, so that there is completely no gas exchange between the inside and the outside of the culture chamber 30 and the culture medium holding chamber 40.

Floating culture of the iPS cells was initiated in the culture chamber 30. Thereafter, once every 2 days, 2 mL of gel culture medium in the culture medium holding chamber 40 was replaced with 2 mL of fresh gel culture medium. 7 to 10 days after initiating culture in the culture chamber 30, the culture medium containing cells within the culture chamber 30 was discharged by the syringe, and the cell masses of iPS cells formed in the gel culture medium were harvested using a filter, washed with PBS, and placed in a Falcon tube. Further, 500 µL of cell dissociation enzyme (TrypLE Select, Thermo Fisher) was added to the cell masses, and the cell masses were incubated for 5 minutes in a CO₂ incubator. Next, the Falcon tube was taken out from the incubator, and 500 µL of cell culture medium was placed in the Falcon tube, and the cell masses were suspended to dissociate the iPS cells into single cells. 2 mL of cell culture medium was added to the Falcon tube, and the Falcon tube was centrifuged at 200g using a centrifuge. After centrifugation, the supernatant in the Falcon tube was removed, and the iPS cells and the gel culture medium were placed in a Falcon tube to prepare a culture medium containing cells. Thereafter, as described above, the culture medium containing cells was injected into the culture chamber 30, and the iPS cells were subjected to floating culture for 7 to 10 days while changing 2 mL of gel culture medium in the culture medium holding chamber 40 once every 2 days.

Thereafter, as described above, passage and floating culture of 7 to 10 days were repeated, and the iPS cells were subjected to floating culture in a sealed culture chamber 30 for a total of 1 month or more.

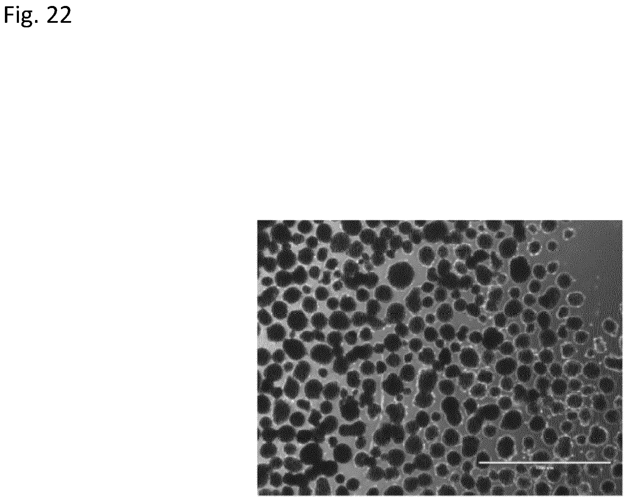

The iPS cells cultured in the culture chamber 30 were observed under microscope, and, as shown in Fig.22, it was confirmed that they formed uniform cell masses.

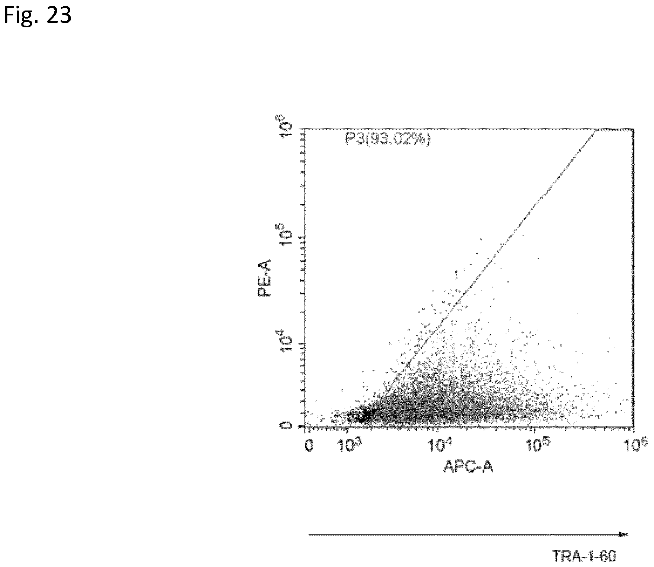

Further, at passage, some of single cell iPS cells were dispensed, and the iPS cells were fixed using 4% paraformaldehyde. Furthermore, the expression level of cell surface antigen TRA-1-60 in the immobilized iPS cells was measured using a flow cytometer. As a result, as shown in Fig.23, the iPS cells at 39 days from initiation of culture were 90% or more TRA-1-60 positive. Thus, it has been shown that, when the vessel is sealed, the stem cells can be cultured for a long period of time while maintaining their pluripotency in an undifferentiated state, without controlling the carbon dioxide concentration in the vessel.

### (Example 7)

The culture medium containing cells was prepared in the same manner as in Example 6. Further, the same cell culture vessel as shown in Fig.2 was prepared. The culture medium containing cells was injected into the culture chamber 30 until the air layer in the culture chamber 30 was completely eliminated. Thereafter, the feed port 231 and the discharge port 331 of the culture chamber 30 were shielded. Further, the culture medium holding chamber 40, the culture medium channel 200, and the culture medium tank 60 were filled with gel culture medium. Thereafter, the inlet and the discharge port of the culture medium tank 60 were shielded. Thus, the inside of the culture chamber 30 and the culture medium holding chamber 40 was sealed, so that there is completely no gas exchange between the inside and the outside of the culture chamber 30 and the culture medium holding chamber 40.

The gel culture medium was circulated through the culture medium holding chamber 40, the culture medium channel 200, and the culture medium tank 60, and floating culture of iPS cells was initiated in the culture chamber 30. Thereafter, once every 2 to 6 days, 10 mL of gel culture medium in the culture medium tank 60 was replaced with 10 mL of fresh gel culture medium. After 7 to 10 days from initiating culture in the culture chamber 30, the culture medium containing cells in the culture chamber 30 was discharged with a syringe, and the same passage treatment as in Example 6 was performed and the culture medium containing cells was injected into the culture chamber 30, and the iPS cells were subjected to floating culture for 7 to 10 days while changing 10 mL of the gel culture medium in the culture medium tank 60 at once every 4 days in the same manner as described above.

Thereafter, as described above, passage and floating culture of 7 to 10 days were repeated, and the iPS cells were subjected to floating culture in a sealed culture chamber 30 for a total of 1 month or more.

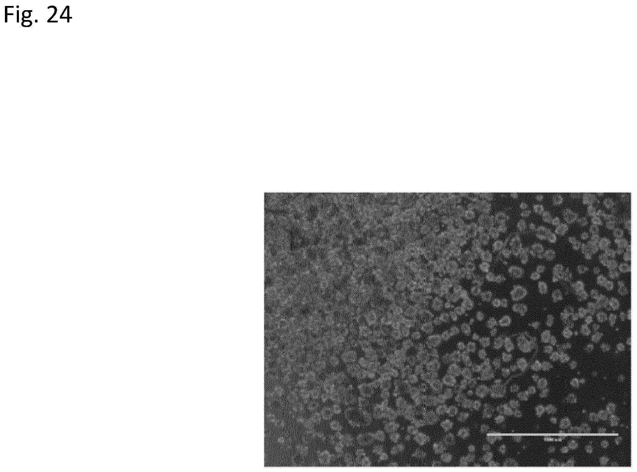

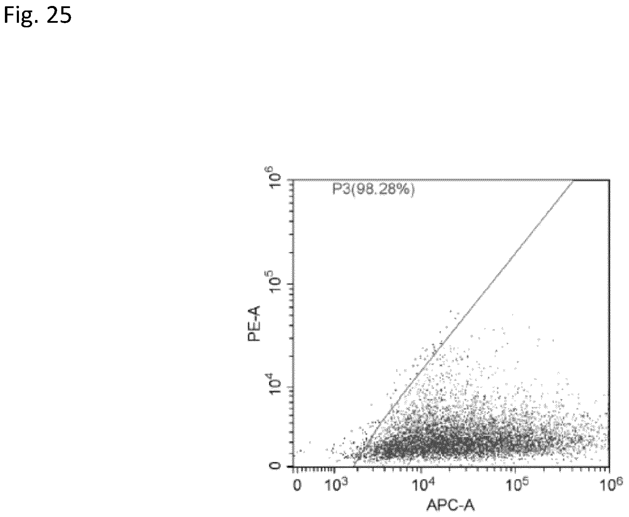

The iPS cells cultured in the culture chamber 30 were observed under microscope, and, as shown in Fig.24, it was confirmed that they formed uniform cell masses. Further, the expression level of cell surface antigen TRA-1-60 in the iPS cells was measured using a flow cytometer in the same manner as in Example 6, as shown in Fig.25, the iPS cells at 15 days from the initiation of culture were almost 100% TRA-1-60 positive.

### (Example 8)

Growth factors were added to the culture medium (StemSpan H3000(R), STEMCELL Technologies Inc.) and deacylated gellan gum was further added to the culture medium to prepare the gel culture medium.

The prepared gel culture medium was placed in a 15 mL tube and the gel culture medium was seeded with 2×10⁵ of hemocytes. Then, the 15 mL tube was placed in a CO₂ incubator and the hemocytes (monocytes) were cultured for 7 days. Thereafter, Sendai virus vectors (CytoTune-iPS2. 0, ID Pharma Co., Ltd.) carrying OCT3/4, SOX2, KLF4, cMYC was added to the gel culture medium to subject the hemocytes to infection with Sendai viruses so that the multiplicity of infection (MOI) is 10.0.

After the Sendai viruses were added to the gel culture medium, 15 mL of gelled stem cell culture medium (DMEM/F12 containing 20%KnockOut SR(R), ThermoFisher SCIENTIFIC) was added to the gel culture medium, and 15 mL of the gel culture medium containing cells infected with Sendai viruses was placed in the culture chamber 30 shown in Fig.20 and Fig.21, and the gel culture medium was injected into the culture medium holding chamber 40. In the same manner as in Example 6, the inside of the culture chamber 30 and the culture medium holding chamber 40 was sealed, so that there is completely no gas exchange between the inside and the outside of the culture chamber 30 and the culture medium holding chamber 40.

Floating culture of the cells transfected with inducing factor in the culture chamber 30 was initiated. Thereafter, once every 2 days, 2 mL of gel culture medium in the culture medium holding chamber 40 was replaced with 2 mL of fresh gel culture medium.

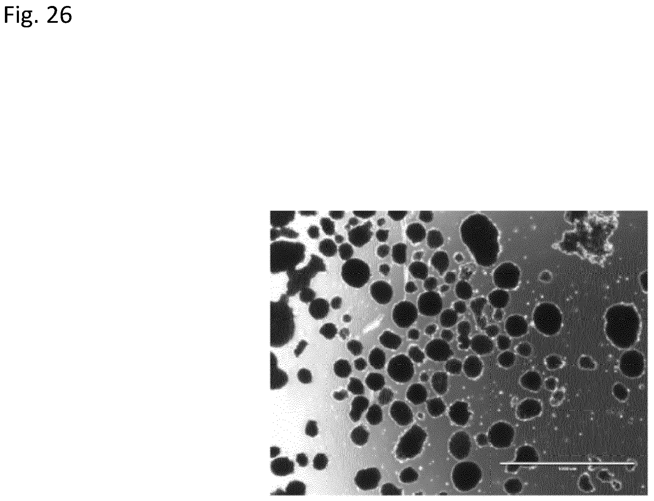

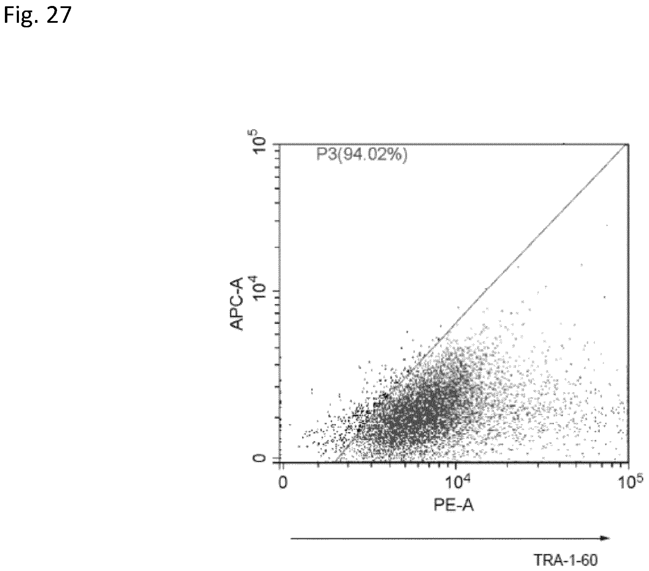

After 15 days, the cells were observed under a microscope, and it was confirmed that they formed ES cell-like colonies, as shown in Fig.26. Further, the cells were immobilized using 4%-paraformaldehyde and the expression level of cell surface antigen TRA-1-60 in the immobilized cells was measured using a flow cytometer, and, as shown in Fig.27, it was confirmed that the expression level was 90% or more TRA-1-60 positive, thus almost completely reprogrammed. Thus, it has been shown that iPS cells can be derived from cells other than stem cells in a completely closed environment without culture medium exchanges and gas exchanges.

### [Reference Signs List]

10: Culture component permeable member, 21: Culture side plate, 22: Culture medium side plate, 30: Culture chamber, 31: Housing, 32: Cover, 33: Plug, 34: Plug, 40: Culture medium holding chamber, 41: Rectifying plate, 51: Introduction fluid machine, 52: Discharge fluid machine, 60: Culture medium tank, 61: Plug, 62: Plug, 70: channel case, 80: Drive unit holding member, 82: Hole, 90: Packing, 110: Semipermeable membrane, 131: Opening, 132: Window, 140: Opening, 145: Discharge block, 151: Pump head, 152: Pump head, 200: Culture medium channel, 231: Feed port, 240: Inlet port, 241: Discharge port, 242: opening, 251: Drive unit, 252: Drive unit, 331: Discharge port, 340: Discharge port, 351: Outside air blocking member for introduction fluid machine, 352: Outside air blocking member for discharge fluid machine, 401: Input device, 402: Output device, 403: Relation storage device, 501: Image processing unit, 511: Contour defining unit, 512: Cell evaluating unit, 513: Statistical processing unit, 514: Density calculating unit, 515: Culture medium evaluating unit.

## Claims

1. A cell culture or induction method comprising subjecting the cells to culture or induction in a sealed system.

2. The method according to claim 1, wherein the induction comprises at least one of reprogramming, initialization, differentiation transformation, differentiation induction, and cell fate reprogramming.

3. The method according to claim 1 or 2, wherein no exchanges of gases take place between the inside and the outside of the sealed system.

4. The method according to any one of claims 1 to 3, further comprising controlling the temperature in the sealed system.

5. The method according to any one of claims 1 to 4, wherein the sealed system is sealed during the culture.

6. The method according to any one of claims 1 to 5, wherein no outside air enters the sealed system when the sealed system is hermetically sealed.

7. The method according to any one of claims 1 to 6, wherein no cells, microorganisms, viruses, and dust outside the sealed system enters the sealed system when the sealed system is hermetically sealed.

8. The method according to any one of claims 1 to 7, wherein no material in the sealed system flows out of the sealed system when the sealed system is hermetically sealed.

9. The method according to any one of claims 1 to 8, wherein at least one of carbon dioxide gas, nitrogen gas, and oxygen gas is not supplied into the sealed system.

10. The method according to any one of claims 1 to 9, wherein the pH of a culture medium in the sealed system is maintained within a predetermined range.

11. The method according to any one of claims 1 to 10, wherein at least a portion of the sealed system is formed by being embedded in a gas impermeable material.

12. The method according to any one of claims 1 to 10, wherein at least a portion of the sealed system is made of a gas impermeable material.

13. The method according to any one of claims 1 to 12, wherein the cells are subjected to culture or induction in the sealed system while the culture medium is supplemented or replaced.

14. The method according to any one of claims 1 to 12, wherein the cells are subjected to culture or induction while the culture medium is circulated in the sealed system.

15. The method according to any one of claims 1 to 14, wherein
the sealed system comprises a culture chamber for culturing the cells
a feed port for supplying fluid into the culture chamber and a discharge port for discharging fluid in the sealed system are provided in culture chamber, and
the feed port and the discharge port are sealable.

16. The method according to claim 15, wherein
a feeder for supplying fluid to the feed port is removable,
a discharger for discharging fluid to the discharge port is removable, and
when fluid is supplied from the feeder into the culture chamber, the fluid in the culture chamber moves into the discharger.

17. The method according to claim 16, wherein air in the culture chamber moves into the discharger when the culture medium is supplied from the feeder into the culture chamber.

18. The method according to claim 16, wherein the culture medium in the culture chamber moves into the discharger when the culture medium is supplied from the feeder into the culture chamber.

19. A method according to claim 17 or 18, wherein said culture medium contains cells.

20. A method according to any one of claims 16 to 19, wherein, in supplying fluid from said feeder into said culture chamber, outside air does not enter into said culture chamber

21. A method according to any one of claims 1 to 20, wherein in said culturing, the carbon dioxide concentration in the sealed system is not controlled.

22. The method according to any one of claims 1 to 21, wherein the carbon dioxide concentration outside the sealed system is not controlled during the culture.

23. The method according to any one of claims 1 to 22, wherein a material in the sealed system moves through a semipermeable membrane in the sealed system during the culture.

24. The method according to any one of claims 1 to 23, wherein
the sealed system comprises a culture chamber for culturing the cells and a channel connected to the culture chamber, and
the culture medium circulates through the culture chamber and the channel.

25. The method according to claim 24, wherein no exchange of gas with the outside occurs in the channel.

26. The method according to claim 24 or 25, wherein the pH of the culture medium in the culture chamber is kept within a predetermined range by the circulation of the culture medium.

27. The method according to any one of claims 1 to 26, wherein the culture is floating culture.

28. The method according to any one of claims 1 to 26, wherein the culture is adherent culture.

29. The method according to any one of claims 1 to 28, wherein the cells are subjected to culture in a gel culture medium within the sealed system.

30. The method according to any one of claims 1 to 28, wherein the cells are subjected to culture in a liquid medium within the sealed system.

31. The method according to any one of claims 1 to 30, wherein the culture medium in the sealed system is stirred.

32. The method according to any one of claims 1 to 30, wherein the culture medium in the sealed system is not stirred.

33. The method according to any one of claims 1 to 32, further comprising passaging the cells.

34. The method according to claim 33, wherein no culture medium is added or replaced between seeding and passage.

35. The method according to claim 33, wherein the culture medium is added or replaced between seeding and passage.

36. The method according to claim 33, wherein no culture medium is added or replaced between passages.

37. The method according to claim 33, wherein the culture medium is added or replaced between passages.

38. The method according to any one of claims 1 to 37, wherein the cells are stem cells.

39. The method according to claim 38, wherein the stem cells are iPS cells, ES cells, or somatic stem cells.

40. The method according to claim 38 or 39, wherein the stem cells maintain an undifferentiated state during the culture.

41. The method according to any one of claims 38 to 40, wherein the stem cells maintain pluripotency during the culture.

42. The method according to any one of claims 1 to 37, wherein the cells are somatic cells.

43. The method according to any one of claims 1 to 37 and 42, wherein the cells are blood cells.

44. The method according to any one of claims 1 to 43, wherein the cells are cells into which an inducing factor has been introduced.

45. The method according to any one of claims 1 to 43, wherein an inducing factor is added to the culture medium in the sealed system, and the inducing factor is introduced into the cells subjected to culture in the sealed system.

46. The method according to claim 44 or 45, wherein the cells are induced to stem cells.

47. The method according to claim 46, wherein the stem cells are iPS cells.

48. The method according to any one of claims 44 to 47, wherein the cells are blood cells.

49. The method according to claim 44 or 45, wherein the cells are induced into another type of cell.

50. The method according to any one of claims 1 to 43, wherein
the cells are blood cell, and
an inducing factor is added to the culture medium in the sealed system, and the inducing factor is introduced into the blood cells subjected to culture in the sealed system to induce the blood cells into iPS cells.

51. The method according to any one of claims 44 to 50, wherein the inducing factor is included in plasmids.

52. The method according to any one of claims 44 to 50, wherein the inducing factor is RNA.

53. The method according to any one of claims 44 to 50, wherein the inducing factor is included in a Sendai virus.

54. A cell culture or induction method comprising
providing a cell culture vessel, including
a culture component permeable member through which a culture component is permeable,
a culture chamber covering one side of the culture component permeable member, and configured to hold a culture medium containing cells and culturing the cells,
a culture medium holding chamber covering the other side of the culture component permeable member, and configured to hold the culture medium; and
subjecting the cells to culture or induction in the culture chamber.

55. The method according to claim 54, wherein the induction comprises at least one of reprogramming, initialization, differentiation transformation, differentiation induction, and cell fate reprogramming.

56. The method according to claim 54 or 55, wherein the interior of the cell culture vessel is closed from the outside.

57. The method according to any one of claims 54 to 56, wherein the pH of the culture medium in the cell culture vessel is maintained within a predetermined range.

58. The method according to any one of claims 54 to 57, wherein the culture is floating culture.

59. The method according to any one of claims 54 to 58, wherein the cells are stem cells.

60. The method according to any one of claims 54 to 58, wherein the cells are somatic cells.

61. The method according to any one of claims 54 to 58 and 60, wherein the cells are blood cells.

62. The method according to any one of claims 54 to 61, wherein the cells are cells into which an inducing factor has been introduced.

63. The method according to any one of claims 54 to 61, wherein the inducing factor is added to the culture medium in the culture chamber, and the inducing factor is introduced into the cells cultured in the culture chamber.

64. The method according to claim 62 or 63, wherein the cells are induced into another type of cell.

65. The method according to any one of claims 54 to 64, wherein the cell culture vessel further comprises a culture side plate provided with openings, which is superimposed on a culture chamber side surface of the culture component permeable member.

66. The method according to any one of claims 54 to 65, wherein the cell culture vessel further comprises a culture medium side plate provided with openings, which is superimposed on a culture medium holding chamber side surface of the culture component permeable member.

67. The method according to claim 65, wherein the culture side plate is dark colored.

68. The method according to claim 65 or 67, further comprising observing the cells or cell masses consisting of the cells against a background of a portion of the culture side plate where the openings are not provided.

69. The method according to claim 65 or 67, further comprising photographing the cells or cell masses consisting of the cells against a background of a portion of the culture side plate where the openings are not provided.

70. The method according to any one of claims 54 to 69, further comprising supplementing or replacing the culture medium in the culture medium holding chamber.
